# EUROPEAN PATENT APPLICATION

(11) **EP 4 564 287 A1**
(43) Date of publication of application: **04.06.2025**
(21) Application number: 23846318.6
(22) Date of filing: 19.07.2023
(51) Int. Cl.: G06T 7/00, G06T 7/11, G16H 50/00

(54) **IMAGE PROCESSING DEVICE, METHOD FOR OPERATION OF IMAGE PROCESSING DEVICE, AND PROGRAM FOR OPERATION OF IMAGE PROCESSING DEVICE**

(30) Priority: 26.07.2022 JP 2022119115
(71) Applicant: FUJIFILM Corporation, Tokyo 106-8620 (JP)
(72) Inventor: NEGISHI, Mitsuru, Ashigarakami-gun, Kanagawa 258-8577 (JP)
(74) Representative: HGF
(86) International application number: PCT/JP2023/026383
(87) International publication number: WO 2024/024587

(57) **Abstract**

An image processing apparatus includes a processor configured to acquire a first specimen image depicting a tissue specimen of a subject; extract, using a machine learning model, first feature amounts from respective first patch images into which the first specimen image is subdivided; determine, based on each of the first feature amounts, whether a morphological abnormality is present in the tissue specimen depicted in a corresponding first patch image of the first patch images; and perform one of manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or soft clustering processing of calculating a degree of belonging of the first patch image determined to have the morphological abnormality in the tissue specimen to each of the plurality of clusters.

## Description

### BACKGROUND OF THE INVENTION

### 1. Field of the Invention

The technique of the present disclosure relates to an image processing apparatus, an operation method of an image processing apparatus, and an operation program of an image processing apparatus.

### 2. Description of the Related Art

US2021/0342570A describes a technique below. By using a machine learning model such as an autoencoder, a feature amount is extracted from a patch image into which a specimen image depicting a tissue specimen of a liver or the like of an animal is subdivided. Based on the extracted feature amount, it is determined whether a morphological abnormality (such as hyperplasia, infiltration, congestion, inflammation, tumor, carcinogenesis, proliferation, bleeding, or glycogen decrease) is present in the tissue specimen depicted in the patch image. Based on the feature amount, the patch image determined to have a morphological abnormality is clustered (hard clustered) into one of a plurality of clusters.

### SUMMARY OF THE INVENTION

To determine whether a morphological abnormality is present, not only image information of a region where the abnormality is present but also image information of the surrounding region are needed. For example, in the case of determining whether bile duct hyperplasia is present as a morphological abnormality, even a specialized pathologist has difficulty determining whether a hyperplastic bile duct region is hyperplastic by observing the region alone and needs to observe a wider field of view including the region and the surrounding region. Therefore, in US2021/0342570A, it is desirable that patch images input to the machine learning model for extracting a feature amount have a size that covers not only the region of the morphological abnormality but also the surrounding region.

However, when the size of the patch images is set as described above, the feature amount is derived not only from the region of the morphological abnormality but also from the surrounding region. Therefore, a distribution of the feature amounts of the patch images determined to have a morphological abnormality is not discrete. When the distribution of the feature amounts is not discrete, it becomes difficult to perform hard clustering by computer processing as described in US2021/0342570A on the patch images determined to have a morphological abnormality.

One embodiment of the technique of the present disclosure provides an image processing apparatus, an operation method of an image processing apparatus, and an operation program of an image processing apparatus capable of clustering patch images of specimen images of which a distribution of feature amounts is not discrete.

An image processing apparatus according to the present disclosure includes a processor configured to acquire a first specimen image depicting a tissue specimen of a subject; extract, using a machine learning model, first feature amounts from respective first patch images into which the first specimen image is subdivided; determine, based on each of the first feature amounts, whether a morphological abnormality is present in the tissue specimen depicted in a corresponding first patch image of the first patch images; and perform one of manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or soft clustering processing of calculating a degree of belonging of the first patch image determined to have the morphological abnormality in the tissue specimen to each of the plurality of clusters.

Preferably, the processor is configured to perform control to display a result of the manual clustering processing or the soft clustering processing.

Preferably, the result is displayed by a plurality of cluster images generated by processing the first specimen image, and the plurality of cluster images are images that enable the plurality of clusters to be identified based on a display format preset for each of the plurality of clusters.

Preferably, the processor is configured to display at least one cluster image among the plurality of cluster images to be superimposed on the first specimen image.

Preferably, the processor is configured to receive, from the user, a designation of the at least one cluster image displayed to be superimposed on the first specimen image.

Preferably, the processor is configured to display statistical information based on the result.

Preferably, the processor is configured to, in the manual clustering processing, reduce a number of dimensions of the first feature amounts to two or three dimensions, display a graph in which the first feature amounts with a reduced number of dimensions are plotted in a two-dimensional space or a three-dimensional space, and receive the designation on the graph.

Preferably, the machine learning model is a model trained using, as labeled training data, second patch images into which second specimen images are subdivided, the second specimen images depicting tissue specimens of a plurality of subjects constituting a control group to which a candidate substance for a medicine is not administered in a past evaluation test of the candidate substance.

Preferably, the labeled training data further includes a patch image depicting the tissue specimen in which the morphological abnormality is present.

Preferably, the machine learning model is a model that performs a task of identifying a type of the morphological abnormality.

Preferably, the processor is configured to acquire information on a distribution of second feature amounts extracted using the machine learning model from the second patch images into which the second specimen images are subdivided; calculate a distance between the distribution and each of the first feature amounts; and perform the determination based on the distance.

An operation method of an image processing apparatus according to the present disclosure includes acquiring a first specimen image depicting a tissue specimen of a subject; extracting, using a machine learning model, first feature amounts from respective first patch images into which the first specimen image is subdivided; determining, based on each of the first feature amounts, whether a morphological abnormality is present in the tissue specimen depicted in a corresponding first patch image of the first patch images; and performing one of manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or soft clustering processing of calculating a degree of belonging of the first patch image determined to have the morphological abnormality in the tissue specimen to each of the plurality of clusters.

An operation program of an image processing apparatus according to the present disclosure causes a computer to execute a process including acquiring a first specimen image depicting a tissue specimen of a subject; extracting, using a machine learning model, first feature amounts from respective first patch images into which the first specimen image is subdivided; determining, based on each of the first feature amounts, whether a morphological abnormality is present in the tissue specimen depicted in a corresponding first patch image of the first patch images; and performing one of manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or soft clustering processing of calculating a degree of belonging of the first patch image determined to have the morphological abnormality in the tissue specimen to each of the plurality of clusters.

The technique of the present disclosure can provide an image processing apparatus, an operation method of an image processing apparatus, and an operation program of an image processing apparatus capable of clustering patch images of specimen images of which a distribution of feature amounts is not discrete.

### BRIEF DESCRIPTION OF THE DRAWINGS

Fig. 1 is a diagram illustrating a process of an evaluation test, first specimen images, and an image processing apparatus;
Fig. 2 is a block diagram illustrating a computer constituting the image processing apparatus;
Fig. 3 is a block diagram illustrating processing units of a central processing unit (CPU) of the image processing apparatus;
Fig. 4 is a diagram illustrating first patch images into which a first specimen image is subdivided;
Fig. 5 is a diagram illustrating how a feature amount extractor extracts first feature amounts from the first patch images;
Fig. 6 is a diagram illustrating how the feature amount extractor is formed;
Fig. 7 is a diagram illustrating processing in a training phase of an autoencoder;
Fig. 8 is a diagram illustrating a past control group and how second patch images for training are formed;
Fig. 9 is a diagram illustrating how the feature amount extractor extracts second feature amounts from the second patch images;
Fig. 10 is a diagram illustrating a graph in which the second feature amounts are plotted in a feature amount space and illustrating second feature amount distribution information;
Fig. 11 is a diagram illustrating a distance between a position of a first feature amount and a representative position of the second feature amounts;
Fig. 12 is a diagram illustrating processing of a determination unit;
Fig. 13 is a diagram illustrating the processing of the determination unit;
Fig. 14 is a diagram illustrating a detailed configuration of a clustering processing unit;
Fig. 15 is a diagram illustrating a target designation screen;
Fig. 16 is a diagram illustrating processing of a dimension reduction unit;
Fig. 17 is a diagram illustrating a cluster designation screen;
Fig. 18 is a diagram illustrating processing of a designation reception unit when a drug discovery staff member designates clusters on the cluster designation screen;
Fig. 19 is a diagram illustrating cluster images and a superimposed image;
Fig. 20 is a diagram illustrating a clustering result display screen;
Fig. 21 is a diagram illustrating a clustering result display screen displaying a superimposed image in which only one cluster image is superimposed on a target first specimen image;
Fig. 22 is a flowchart illustrating a processing procedure of the image processing apparatus;
Fig. 23 is a flowchart illustrating the processing procedure of the image processing apparatus;
Fig. 24 is a diagram illustrating processing of a dimension reduction unit according to a modification;
Fig. 25 is a diagram illustrating a cluster designation screen according to the modification;
Fig. 26 is a diagram illustrating a detailed configuration of a clustering processing unit according to a first embodiment_2;
Fig. 27 is a diagram illustrating processing of a degree-of-belonging calculation unit and a clustering information generation unit;
Fig. 28 is a flowchart illustrating a processing procedure of the image processing apparatus according to the first embodiment_2;
Fig. 29 is a diagram illustrating a clustering result display screen according to the first embodiment_2;
Fig. 30 is a diagram illustrating a clustering result display screen displaying statistical information;
Fig. 31 is a diagram illustrating processing in a training phase of an autoencoder according to a third embodiment;
Fig. 32 is a diagram illustrating a past administration group and how third patch images for training are formed;
Fig. 33 is a diagram illustrating how a feature amount extractor according to a fourth embodiment is formed;
Fig. 34 is a diagram illustrating a fifth embodiment that handles a specimen image obtained by imaging a slide specimen on which tissue specimens of a plurality of types of organs are placed; and
Fig. 35 is a diagram illustrating how a feature amount extractor for heart specimens extracts first feature amounts from first patch images into which a heart specimen is subdivided.

### DESCRIPTION OF THE PREFERRED EMBODIMENTS

### [First Embodiment_1]

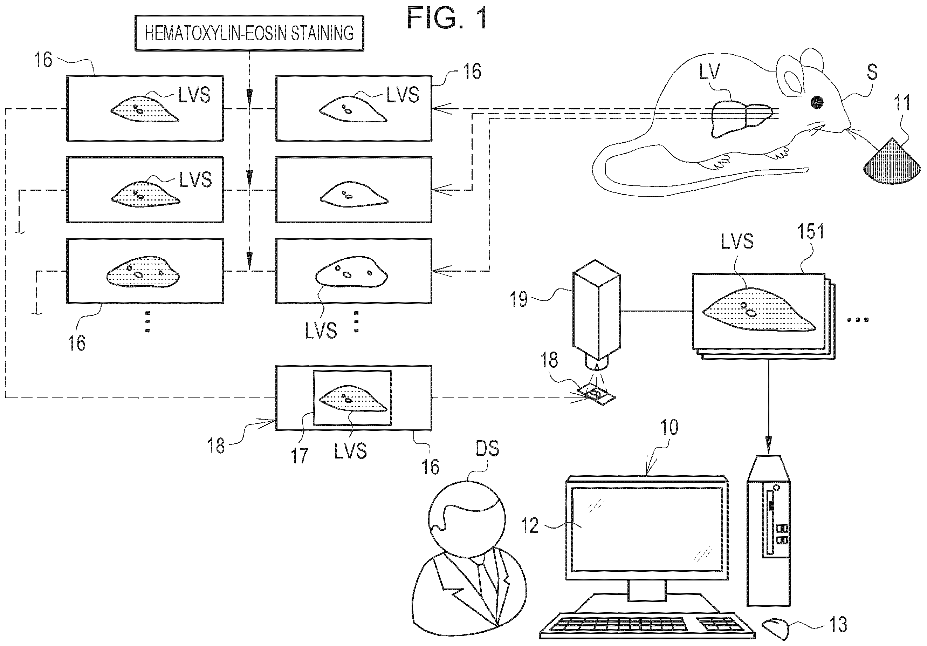

As illustrated in Fig. 1 as an example, an image processing apparatus 10 of the present disclosure is used to evaluate the efficacy and toxicity of a candidate substance 11 for a medicine. The image processing apparatus 10 is, for example, a desktop personal computer, and includes a display 12 that displays various screens and an input device 13 such as a keyboard, a mouse, a touch panel, and/or a microphone for audio input. The image processing apparatus 10 is installed, for example, in a pharmaceutical development facility and is operated by a drug discovery staff member DS involved in pharmaceutical development at the pharmaceutical development facility. The drug discovery staff member DS includes a pathologist. The drug discovery staff member DS is an example of a "user" according to the technique of the present disclosure.

First specimen images 151 are input to the image processing apparatus 10. The first specimen images 151 are images used for evaluating the efficacy and toxicity of the candidate substance 11. The first specimen images 151 are each generated, for example, in a procedure below. First, a subject S such as a rat prepared for evaluation of the candidate substance 11 is autopsied to collect a plurality of tissue specimens (hereinafter, referred to as liver specimens) LVS of cross sections of an organ, in this case a liver LV, of the subject S. Next, the collected liver specimens LVS are each placed on a slide glass 16. Then, the liver specimens LVS are stained, in this case, with hematoxylin-eosin stain. Subsequently, each of the stained liver specimens LVS is covered with a cover glass 17 to complete a slide specimen 18. Then, the slide specimen 18 is placed at an imaging device 19 such as a digital optical microscope, and the imaging device 19 captures the first specimen image 151. The first specimen image 151 thus obtained is assigned a subject identification data (ID) for uniquely identifying the subject S, a specimen image ID for uniquely identifying the first specimen image 151, an imaging date and time, etc. Note that the tissue specimen is also referred to as a tissue section. In addition, the staining may be staining using hematoxylin dye alone, staining using nuclear fast red dye, or the like.

An administration group and a control group will be described. The administration group is constituted by a plurality of subjects S to which the candidate substance 11 is administered. In contrast to the administration group, the control group is constituted by a plurality of subjects S to which the candidate substance 11 is not administered. The number of subjects S constituting the administration group and the number of subjects S constituting the control group are both, for example, approximately 5 to 10. The subjects S constituting the administration group and the subjects S constituting the control group are the subjects S having the same attributes and placed under the same rearing environment. The same attributes refer to, for example, the same week age, the same sex, and/or the like. The same attributes also include a same week age composition ratio and/or the same sex composition ratio (such as five males and five females). The same rearing environment refers to, for example, the same food that is given, the same temperature and humidity in a rearing space, the same rearing space size, and/or the like. The term "same" in the same rearing environment encompasses not only being completely the same but also being the same including a margin of error which is generally allowed in the technical field to which the technique of the present disclosure pertains and which is of a degree not inconsistent with the gist of the technique of the present disclosure.

In the administration group, there are a plurality of subgroups with different doses of the candidate substance 11. For example, the dose of the candidate substance 11 is made different in three levels such as a high-dose group, a medium-dose group, and a low-dose group. This makes it possible to inspect the effect of the different dose of the candidate substance 11 on the subjects S.

The first specimen images 151 may be images depicting the liver specimen LVS of the subject S of the administration group or images depicting the liver specimen LVS of the subject S of the control group. Fig. 1 illustrates a case where the first specimen images 151 are images of the liver specimen LVS of the subject S of the administration group.

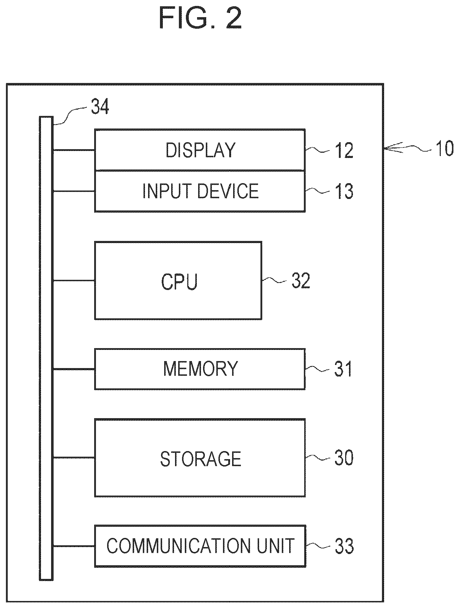

As illustrated in Fig. 2 as an example, a computer constituting the image processing apparatus 10 includes a storage 30, a memory 31, a central processing unit (CPU) 32, and a communication unit 33, in addition to the display 12 and the input device 13 described above. These are interconnected via a bus line 34.

The storage 30 is a hard disk drive built in or connected, via a cable or network, to the computer constituting the image processing apparatus 10. Alternatively, the storage 30 may be a disk array of a plurality of linked hard disk drives. The storage 30 stores a control program such as an operating system, various application programs, and various kinds of data associated with these programs. Note that a solid-state drive may be used instead of the hard disk drive.

The memory 31 is a work memory for the CPU 32 to execute processing. The CPU 32 loads the program stored in the storage 30 into the memory 31 and executes processing according to the program. Thus, the CPU 32 comprehensively controls each part of the computer. The CPU 32 is an example of a "processor" according to the technique of the present disclosure. The memory 31 may be built in the CPU 32. The communication unit 33 controls transmission of various kinds of information with an external device such as the imaging device 19.

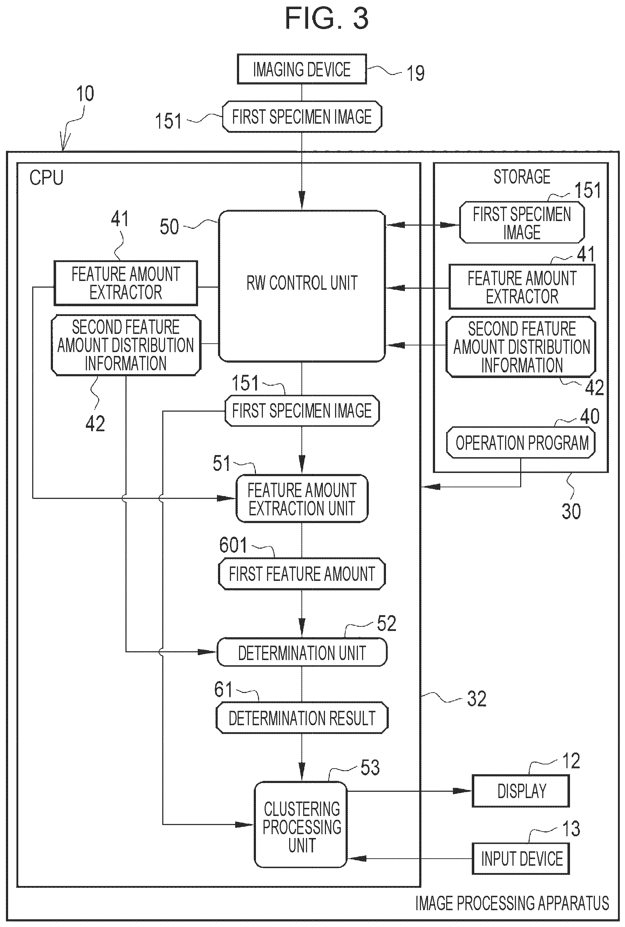

As illustrated in Fig. 3 as an example, the storage 30 of the image processing apparatus 10 stores an operation program 40. The operation program 40 is an application program for causing the computer to function as the image processing apparatus 10. That is, the operation program 40 is an example of an "operation program of an image processing apparatus" according to the technique of the present disclosure. The storage 30 also stores a feature amount extractor 41, second feature amount distribution information 42, and so on. The feature amount extractor 41 is an example of a "machine learning model" according to the technique of the present disclosure. In addition, the second feature amount distribution information 42 is an example of "information on a distribution of second feature amounts" according to the technique of the present disclosure.

When the operation program 40 is started, the CPU 32 of the computer constituting the image processing apparatus 10 functions, in cooperation with the memory 31 and the like, as a read/write (hereinafter abbreviated as RW) control unit 50, a feature amount extraction unit 51, a determination unit 52, and a clustering processing unit 53.

The RW control unit 50 controls storing of various kinds of data in the storage 30 and reading out of various kinds of data in the storage 30. For example, the RW control unit 50 stores the first specimen images 151 from the imaging device 19 in the storage 30. Since a plurality of first specimen images 151 are actually obtained from a single subject S, the plurality of first specimen images 151 are stored in the storage 30 for the single subject S.

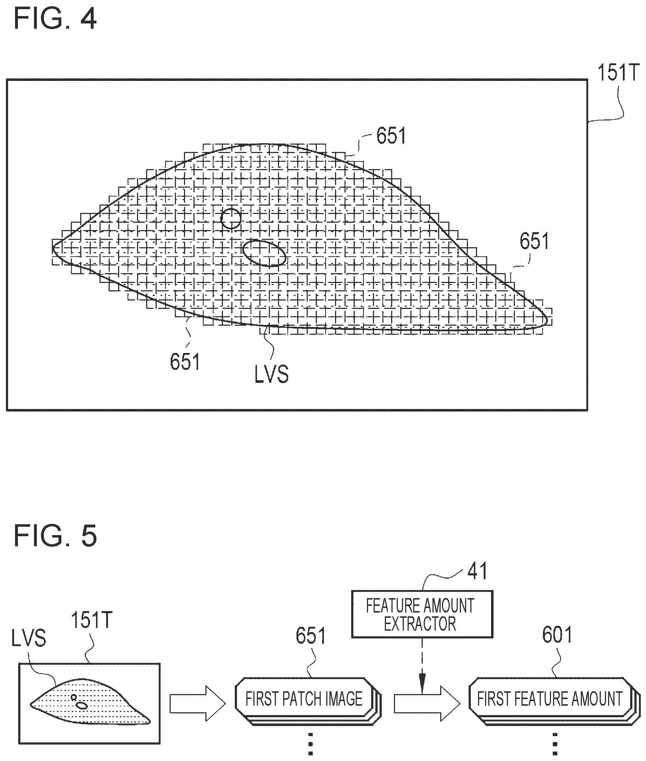

The RW control unit 50 reads out, from the storage 30, and thus acquires the first specimen images 151 corresponding to a designation made by the drug discovery staff member DS through the input device 13. The RW control unit 50 outputs the read out first specimen images 151 to the feature amount extraction unit 51 and the clustering processing unit 53. The first specimen images 151 output from the RW control unit 50 to the feature amount extraction unit 51 and the like are subjected to determination as to whether a morphological abnormality is present in the liver specimen LVS. Hereinafter, the first specimen image 151 that is subjected to determination as to whether a morphological abnormality is present in the liver specimen LVS is referred to as a target first specimen image 151T (see Fig. 4 and others).

The RW control unit 50 reads out the feature amount extractor 41 from the storage 30, and outputs the read out feature amount extractor 41 to the feature amount extraction unit 51. The RW control unit 50 also reads out, from the storage 30, and thus acquires the second feature amount distribution information 42. The RW control unit 50 outputs the read out second feature amount distribution information 42 to the determination unit 52.

The feature amount extraction unit 51 extracts, using the feature amount extractor 41, a first feature amount 601 from the target first specimen image 151T. The feature amount extraction unit 51 outputs the first feature amount 601 to the determination unit 52.

The determination unit 52 determines whether a morphological abnormality is present in the liver specimen LVS depicted in the target first specimen image 151T, based on the first feature amount 601 and the second feature amount distribution information 42. A morphological abnormality refers to a lesion not observed in a normal liver specimen LVS, for example, hyperplasia, infiltration, congestion, inflammation, tumor, carcinogenesis, proliferation, bleeding, glycogen decrease, or the like. The determination unit 52 outputs a determination result 61 indicating whether a morphological abnormality is present in the liver specimen LVS depicted in the target first specimen image 151T, to the clustering processing unit 53.

The clustering processing unit 53 performs manual clustering processing in the present embodiment. The manual clustering processing will be described later.

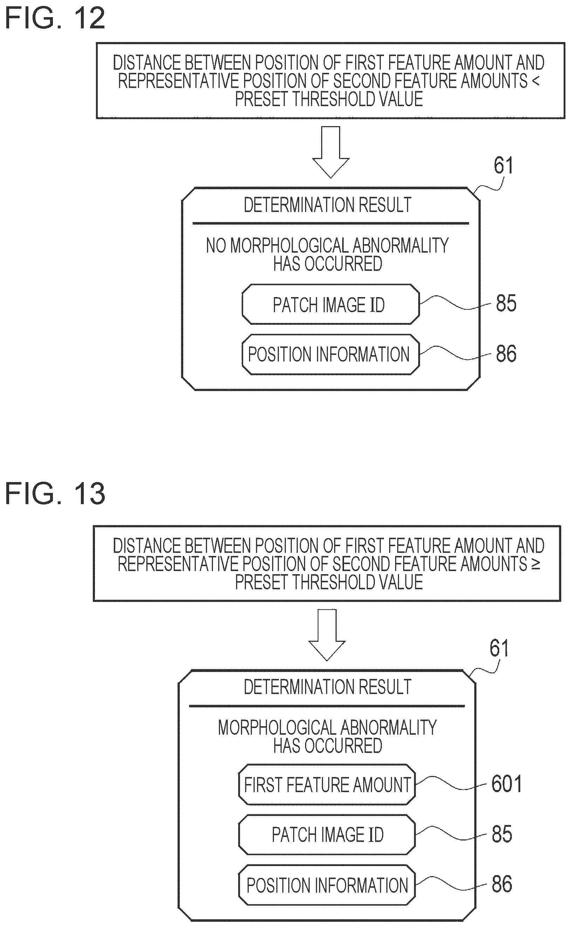

As illustrated in Fig. 4 as an example, the feature amount extraction unit 51 recognizes the liver specimen LVS depicted in the target first specimen image 151T using a well known image recognition technique, and subdivides the recognized liver specimen LVS into a plurality of first patch images 651. The first patch images 651 have a preset size that can be handled by the feature amount extractor 41. In addition, the first patch images 651 have a size that covers not only a region of a morphological abnormality but also the surrounding region. The feature amount extraction unit 51 assigns a patch image ID 85 (see Fig. 12 and others) to each of the first patch images 651. In addition, the feature amount extraction unit 51 associates, with the patch image ID 85, information indicating which location of the first specimen image 151T the first patch image 651 is cut out from, that is, position information 86 (see Fig. 12 and others) of the first patch image 651. In Fig. 4, each first patch image 651 does not have an overlapping region with another first patch image 651. However, each first patch image 651 may partially overlap another first patch image 651.

As illustrated in Fig. 5 as an example, the feature amount extraction unit 51 extracts, using the feature amount extractor 41, the first feature amount 601 for each of the plurality of first patch images 651 into which the target first specimen image 151T is subdivided. Therefore, the number of first feature amounts 601 is equal to the number of first patch images 651.

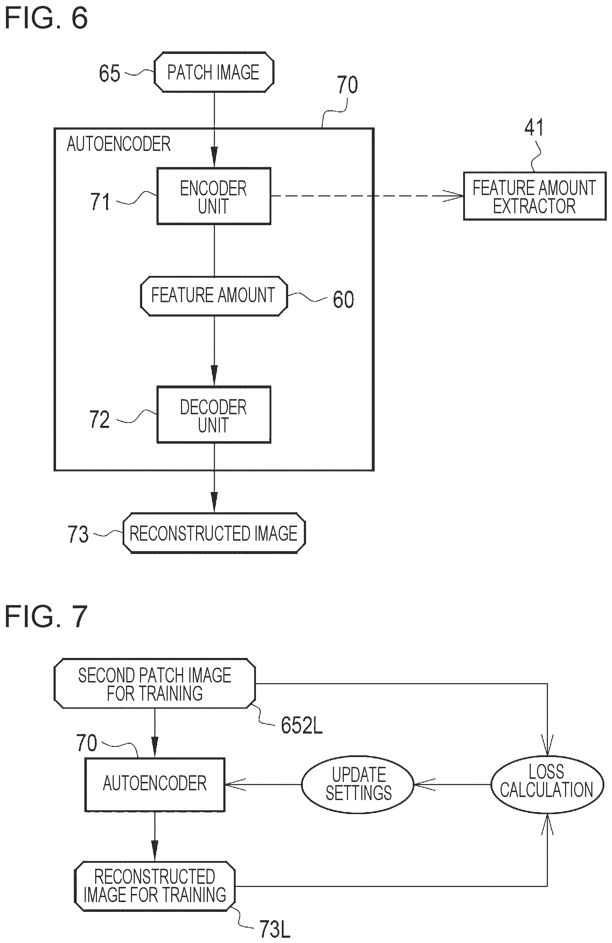

As illustrated in Fig. 6 as an example, an encoder unit 71 of an autoencoder 70 is repurposed as the feature amount extractor 41. The autoencoder 70 has a decoder unit 72 in addition to the encoder unit 71. A patch image 65, such as the first patch image 651, is input to the encoder unit 71. The encoder unit 71 converts the patch image 65 into a feature amount 60. The encoder unit 71 passes the feature amount 60 to the decoder unit 72. The decoder unit 72 generates a reconstructed image 73 of the patch image 65 from the feature amount 60.

As is well known, the encoder unit 71 has layers such as a convolution layer that performs convolution processing using a filter and a pooling layer that performs pooling processing such as maximum value pooling processing. The same applies to the decoder unit 72. The encoder unit 71 extracts the feature amount 60 by repeatedly performing the convolution processing using the convolutional layer and the pooling processing using the pooling layer multiple times on the input patch image 65. The extracted feature amount 60 represents characteristics of the shape and texture of the liver specimen LVS depicted in the patch image 65.

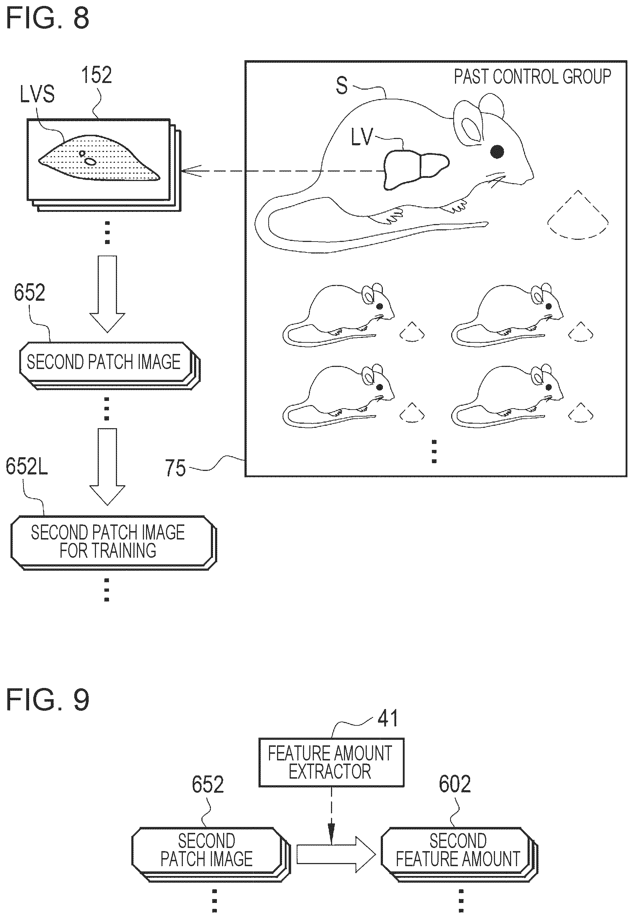

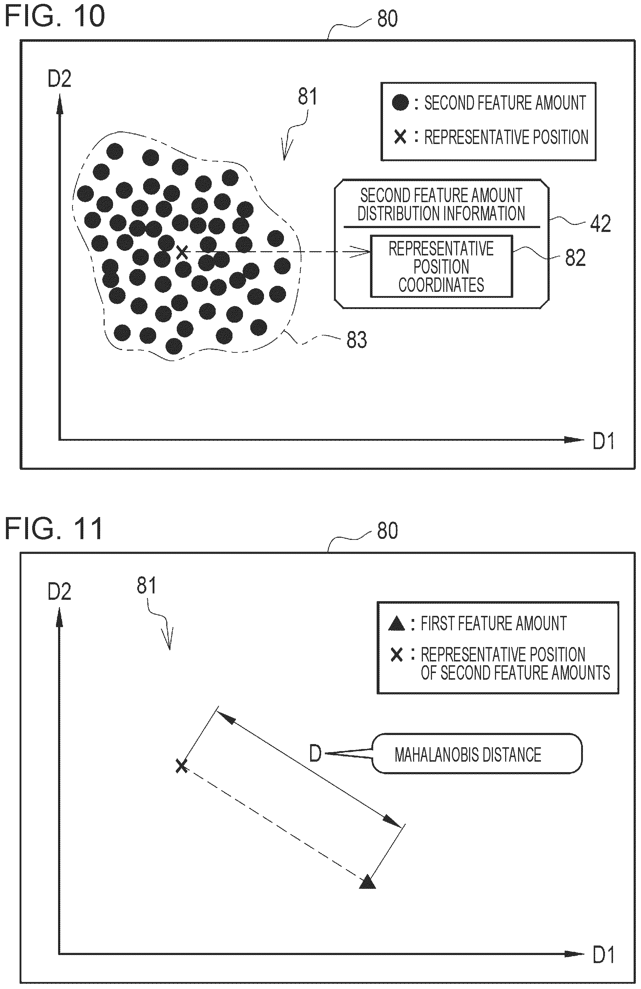

The feature amount 60 is a set of a plurality of numerical values. In other words, the feature amount 60 is multidimensional data. The number of dimensions of the feature amount 60 is, for example, 512, 1024, or 2048. The first feature amount 601 and a second feature amount 602 (see Fig. 9) to be described later have the same number of dimensions and can be compared with each other in an identical feature amount space 81 (see Fig. 10 and others).

As illustrated in Fig. 7 as an example, the autoencoder 70 receives, as an input, a second patch image 652L for training and is trained on the second patch image 652L for training in a training phase before the encoder unit 71 is repurposed as the feature amount extractor 41. For the second patch image 652L for training, the autoencoder 70 outputs a reconstructed image 73L for training. Based on the second patch image 652L for training and the reconstructed image 73L for training, a loss calculation of the autoencoder 70 is performed using a loss function. Then, update settings of the various coefficients (such as coefficients of the filter in the convolutional layer) of the autoencoder 70 are made based on the result of the loss calculation, and the autoencoder 70 is updated in accordance with the update settings. The second patch image 652L for training is an example of "labeled training data" according to the technique of the present disclosure.

In the training phase of the autoencoder 70, the series of processing of inputting the second patch image 652L for training to the autoencoder 70, outputting the reconstructed image 73L for training from the autoencoder 70, performing the loss calculation, making the update settings, and updating the autoencoder 70 is repeatedly performed while the second patch image 652L for training is replaced. The repetition of the series of processing is terminated when the reconstruction accuracy from the second patch image 652L for training to the reconstructed image 73L for training reaches a predetermined set level. The encoder unit 71 of the autoencoder 70 of which the reconstruction accuracy has reached the set level is stored in the storage 30 of the image processing apparatus 10 as the feature amount extractor 41. Note that training may be terminated after the series of processing is repeated a set number of times, regardless of the reconstruction accuracy from the second patch image 652L for training to the reconstructed image 73L for training.

The training of the autoencoder 70 may be performed by the image processing apparatus 10 or by an apparatus different from the image processing apparatus 10. In the latter case, the feature amount extractor 41 is transmitted from the different apparatus to the image processing apparatus 10, and the RW control unit 50 stores the feature amount extractor 41 in the storage 30.

As illustrated in Fig. 8 as an example, the second patch image 652L for training is supplied from among a plurality of second patch images 652 into which the second specimen images 152 are subdivided. The second specimen images 152 are images depicting the liver specimens LVS of the subjects S in a past control group 75. The past control group 75 is constituted by a plurality of subjects S to which the candidate substance was not administered in past evaluation tests. Therefore, the number of subjects S constituting the past control group 75 is much larger than the number of subjects S constituting the administration group and the control group, and ranges approximately from several hundreds to several thousands, for example. Since a plurality of second specimen images 152 are obtained from a single subject S just like the first specimen images 151, as many second specimen images 152 as a product of the number of second specimen images 152 obtained the single subject S and the number of subjects S are obtained from the past control group 75.

Next, how the second feature amount distribution information 42 is formed will be described. First, as illustrated in Fig. 9 as an example, using the feature amount extractor 41, a plurality of second feature amounts 602 are extracted from a plurality of second patch images 652 that are based on the plurality of second specimen images 152 obtained from all the subjects S constituting the past control group 75.

In a graph 80 illustrated in Fig. 10 as an example, the plurality of second feature amounts 602 extracted in Fig. 9 are plotted in the feature amount space 81. The second feature amount distribution information 42 includes coordinates (hereinafter, referred to as representative position coordinates) 82 of a representative position of the second feature amounts 602, indicated by a cross mark, in the feature amount space 81. The representative position is, for example, the center point or the average point of a distribution 83 of the second feature amounts 602. Note that in Fig. 10, for the sake of explanation, the dimensions of the feature amount space 81 are two dimensions having D1 and D2 axes. However, the actual dimensions of the feature amount space 81 are 512 dimensions or the like as described before. Similarly, in Fig. 11 below, for the sake of explanation, the dimensions of the feature amount space 81 are represented in two dimensions.

As in training of the autoencoder 70, the second feature amount distribution information 42 may be created by the image processing apparatus 10 or by an apparatus different from the image processing apparatus 10. In the latter case, the second feature amount distribution information 42 is transmitted from the different apparatus to the image processing apparatus 10, and the RW control unit 50 stores the second feature amount distribution information 42 in the storage 30. Additionally, the second feature amount distribution information 42 may be the plurality of second feature amounts 602 themselves. In this case, the representative position coordinates 82 are derived by the image processing apparatus 10.

As illustrated in Fig. 11 as an example, the determination unit 52 calculates a distance D in the feature amount space 81 between the representative position of the second feature amounts 602, which is represented by the representative position coordinates 82 of the second feature amount distribution information 42, and the position of the first feature amount 601. The determination unit 52 calculates the distance D for each of the plurality of first feature amounts 601 extracted from the plurality of first patch images 651 into which a single target first specimen image 151T is subdivided. The distance D is a Mahalanobis distance. Note that, as the distance D, any one of the average value, median value, or maximum value of Euclidean distances between the position of the first feature amount 601 and the positions of k-nearest neighbor samples in the distribution 83 of the second feature amounts 602 may be calculated. Alternatively, instead of the distance D, a value obtained by subtracting a cosine similarity between a vector representing the representative position of the second feature amounts 602 and a vector representing the position of the first feature amount 601 from 1.0 may be calculated. The cosine similarity takes a value from -1.0 to 1.0. A larger value indicates a greater similarity in the direction of the vectors.

As illustrated in Fig. 12 as an example, the determination unit 52 compares the calculated distance D with a preset threshold value. If the distance D is less than the threshold value, the determination unit 52 determines that no morphological abnormality is present in the liver specimen LVS depicted in the first patch image 651. The determination unit 52 outputs the determination result 61 indicating that no morphological abnormality is present in the liver specimen LVS depicted in the first patch image 651. The determination result 61 in this case includes the patch image ID 85 and the position information 86.

On the other hand, as illustrated in Fig. 13 as an example, if the distance D is equal to or greater than the threshold value, the determination unit 52 determines that a morphological abnormality is present in the liver specimen LVS depicted in the first patch image 651. The determination unit 52 outputs the determination result 61 indicating that a morphological abnormality is present in the liver specimen LVS depicted in the first patch image 651. The determination result 61 in this case includes the first feature amount 601 in addition to the patch image ID 85 and the position information 86. Note that the drug discovery staff member DS may be allowed to change the setting of the threshold value. Instead of the distance D, the determination unit 52 may determine whether a morphological abnormality is present in the liver specimen LVS depicted in the first patch image 651, based on comparison of the aforementioned cosine similarity with a threshold value.

As illustrated in Figs. 8 and 9, the second feature amounts 602 are feature amounts extracted from the second patch images 652 into which the second specimen images 152 depicting the liver specimens LVS of the subjects S in the past control group 75 are subdivided. Since the candidate substance was not administered to the subjects S in the past control group 75, at least any morphological abnormality due to the toxicity of the candidate substance is not present in the liver specimens LVS depicted in the second specimen images 152. Accordingly, the representative position of the second feature amounts 602 is regarded as the representative position of feature amounts of specimen images depicting a normal liver specimen LVS. Therefore, the distance D between the position of the first feature amount 601 and the representative position of the second feature amounts 602 serves as an indicator of how much the liver specimen LVS depicted in the first patch image 651 deviates from the normal liver specimen LVS. Thus, for the first patch image 651 for which the distance D is less than the threshold value, the determination unit 52 determines that no morphological abnormality is present in the liver specimen LVS as the liver specimen LVS does not deviate from the normal liver specimen LVS. On the other hand, for the first patch image 651 for which the distance D is equal to or greater than the threshold value, the determination unit 52 determines that a morphological abnormality is present in the liver specimen LVS as the liver specimen LVS deviates from the normal liver specimen LVS.

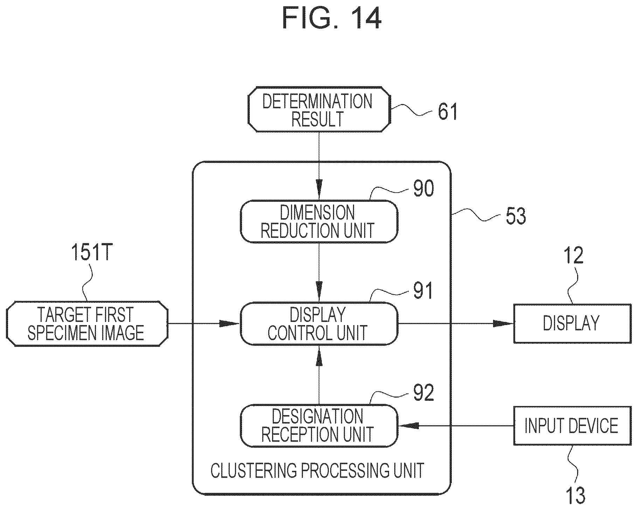

As illustrated in Fig. 14 as an example, the clustering processing unit 53 includes a dimension reduction unit 90, a display control unit 91, and a designation reception unit 92. The dimension reduction unit 90, the display control unit 91, and the designation reception unit 92 perform manual clustering processing described below. The determination result 61 from the determination unit 52 is input to the dimension reduction unit 90. The target first specimen image 151T from the RW control unit 50 is input to the display control unit 91. The display control unit 91 performs control to display various screens on the display 12. The designation reception unit 92 receives various designations made by the drug discovery staff member DS through the input device 13.

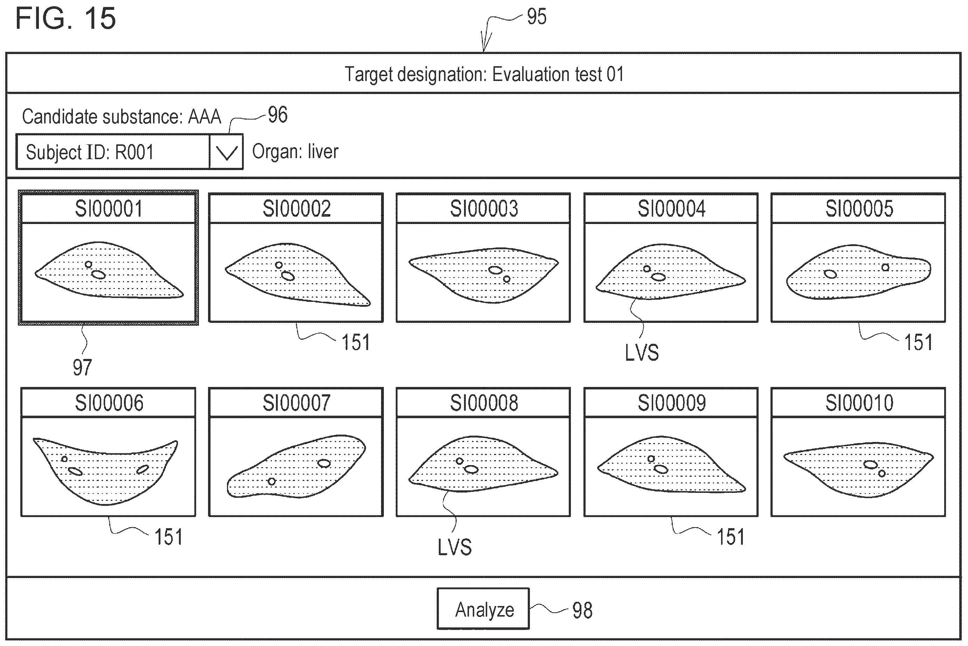

As illustrated in Fig. 15 as an example, the display control unit 91 performs control to display a target designation screen 95 on the display 12. The display control unit 91 displays the target designation screen 95 when the drug discovery staff member DS gives an instruction to display the first specimen images 151 through the input device 13 and the designation reception unit 92 receives this display instruction. The target designation screen 95 displays the plurality of first specimen images 151 in an arranged manner. The plurality of first specimen images 151 are the first specimen images 151 obtained from a single subject S among the plurality of subjects S constituting the administration group. Fig. 15 illustrates an example in which ten first specimen images 151 with specimen image IDs "SI00001" to "SI00010", obtained from the subject S with the subject ID "R001", are displayed in an arranged manner. The subject ID is provided as a pull-down menu 96, which allows switching of the subject S of which the first specimen images 151 are displayed on the target designation screen 95.

The target designation screen 95 is a screen for designating a single target first specimen image 151T from among the plurality of first specimen images 151. The target designation screen 95 is provided with a single selection frame 97 movable among the plurality of first specimen images 151. The target designation screen 95 is also provided with an analyze button 98 at a lower portion thereof. The drug discovery staff member DS places the selection frame 97 at a desired first specimen image 151, and then selects the analyze button 98. As a result, the first specimen image 151 at which the selection frame 97 is placed is set as the target first specimen image 151T, the feature amount extraction unit 51 extracts the first feature amount 601 and the determination unit 52 determines whether a morphological abnormality is present.

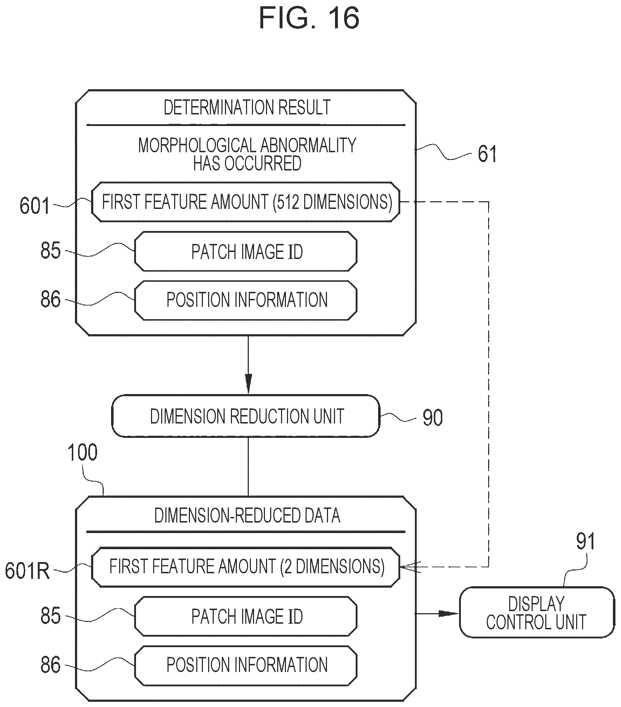

As illustrated in Fig. 16 as an example, the dimension reduction unit 90 reduces the number of dimensions of the first feature amount 601 included in the determination result 61 for the first patch image 651 determined to have a morphological abnormality in the liver specimen LVS, to two dimensions. The dimension reduction unit 90 generates dimension-reduced data 100 based on the determination result 61. The dimension-reduced data 100 includes a dimension-reduced first feature amount 601R, the patch image ID 85, and the position information 86. The dimension reduction unit 90 outputs the dimension-reduced data 100 to the display control unit 91. Note that as a dimension reduction method, principal component analysis (PCA), t-distributed stochastic neighbor embedding (t-SNE), uniform manifold approximation and projection (UMAP), or the like can be used.

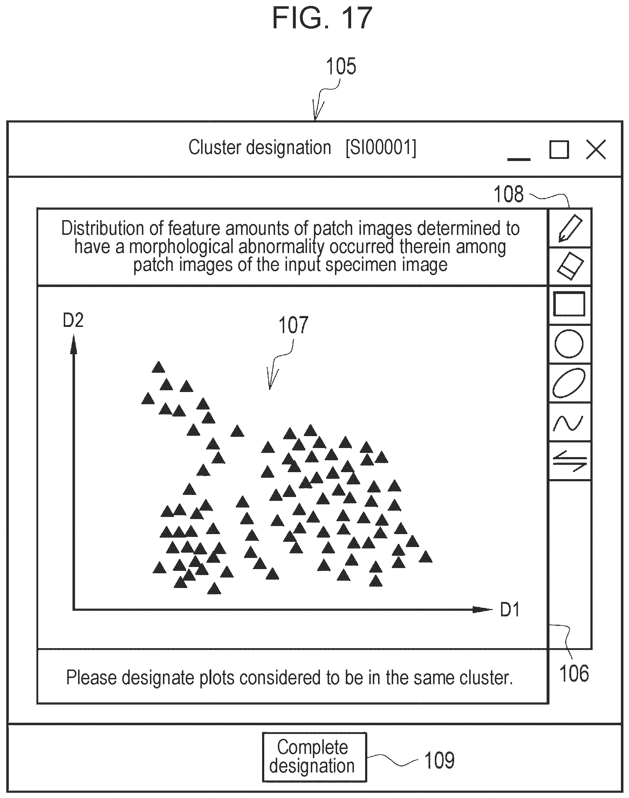

When receiving the dimension-reduced data 100 from the dimension reduction unit 90, the display control unit 91 performs control to display a cluster designation screen 105, illustrated in Fig. 17 as an example, on the display 12. The cluster designation screen 105 displays a graph 106. The graph 106 is a graph in which dimension-reduced two-dimensional first feature amounts 601R included in the dimension-reduced data 100 are plotted in a two-dimensional feature amount space 107.

As described above, the first patch image 651 has a size that covers not only a region of a morphological abnormality but also the surrounding region. Therefore, the distribution of the first feature amounts 601R is not discrete.

A cluster designation toolbar 108 is provided to the right of the graph 106. The cluster designation toolbar 108 is a toolbar to which icons of various tools are collected. The various tools are used by the drug discovery staff member DS to designate, on the graph 106, which cluster each first patch image 651 determined to have a morphological abnormality in the liver specimen LVS belongs to. The tools include a tool for enclosing a group of the plots of the first feature amounts 601R, which is considered to be a cluster, using a rectangular, circular, elliptic, or free curve. The tools also include a tool for erasing the rectangular, circular, elliptic, or free curve enclosing the plots of the first feature amounts 601R or a tool for changing the designated cluster.

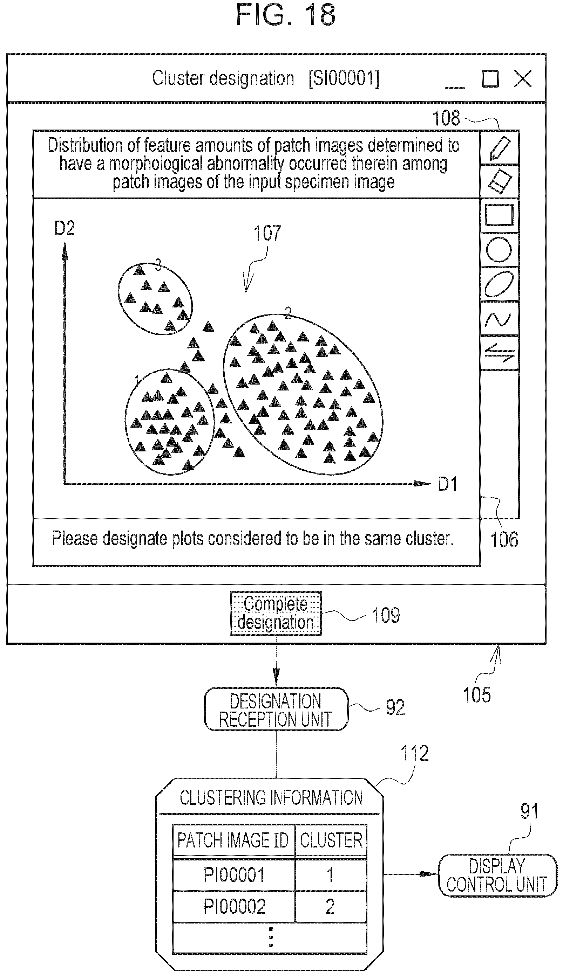

As illustrated in Fig. 18 as an example, the drug discovery staff member DS uses the various tools of the cluster designation toolbar 108 to designate which cluster each first patch image 651 belongs to on the graph 106. Fig. 18 illustrates a case where three clusters, namely, clusters 1, 2, and 3, are designated by enclosing the clusters with respective ellipses. As illustrated, some plots of the first feature amounts 601R do not belong to any of the clusters.

The cluster designation screen 105 is provided with a complete designation button 109 at a lower portion thereof. After designating the clusters on the graph 106, the drug discovery staff member DS selects the complete designation button 109. As a result, the designation reception unit 92 receives the designation made by the drug discovery staff member DS and indicating which cluster each first patch image 651 determined to have a morphological abnormality in the liver specimen LVS belongs to.

The designation reception unit 92 generates clustering information 112, based on the received designation. The clustering information 112 is information in which, for each patch image ID 85, the cluster to which the corresponding first patch image 651 belongs is registered. The first patch image 651 of which the plot of the first feature amount 601R does not belong to any of the clusters is not registered in the clustering information 112. The designation reception unit 92 outputs the clustering information 112 to the display control unit 91.

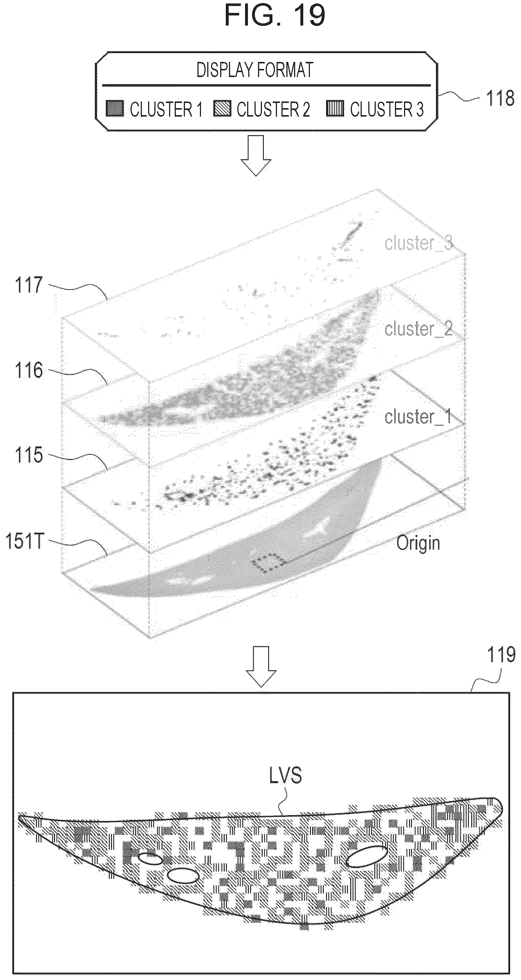

As illustrated in Fig. 19 as an example, the display control unit 91 generates cluster images 115, 116, and 117 by processing the target first specimen image 151T in accordance with the clustering information 112 received from the designation reception unit 92. The cluster image 115 corresponds to the cluster 1 illustrated in Fig. 18. The cluster image 116 corresponds to the cluster 2 illustrated in Fig. 18. The cluster image 117 corresponds to the cluster 3 illustrated in Fig. 18.

The display control unit 91 generates the cluster images 115 to 117 in accordance with a display format 118 preset for each of the clusters 1 to 3. For example, the display format 118 indicates that the cluster 1, the cluster 2, and the cluster 3 are displayed in indigo, yellow-green, and gray, respectively. The display control unit 91 generates the cluster image 115 by filling the positions (which can be identified from the position information 86) of the first patch images 651 with the patch image IDs 85 registered in association with the cluster 1 in the clustering information 112, in indigo in the target first specimen image 151T. Likewise, the display control unit 91 generates the cluster image 116 by filling the positions of the first patch images 651 with the patch image IDs 85 registered in association with the cluster 2 in the clustering information 112, in yellow-green in the target first specimen image 151T. The display control unit 91 further generates the cluster image 117 by filling the positions of the first patch images 651 with the patch image IDs 85 registered in association with the cluster 3 in the clustering information 112, in gray in the target first specimen image 151T. By changing the display colors in this manner, the cluster images 115 to 117 become images that enable identification of the clusters 1 to 3. The cluster images 115 to 117 are an example of a "result of manual clustering processing" according to the technique of the present disclosure. Note that the drug discovery staff member DS may be allowed to change the setting of the display format 118 in any manner.

The display control unit 91 generates a superimposed image 119 in which at least one of the cluster images 115 to 117 is superimposed on the target first specimen image 151T. Fig. 19 illustrates the superimposed image 119 in which all the cluster images 115 to 117 are superimposed on the target first specimen image 151T.

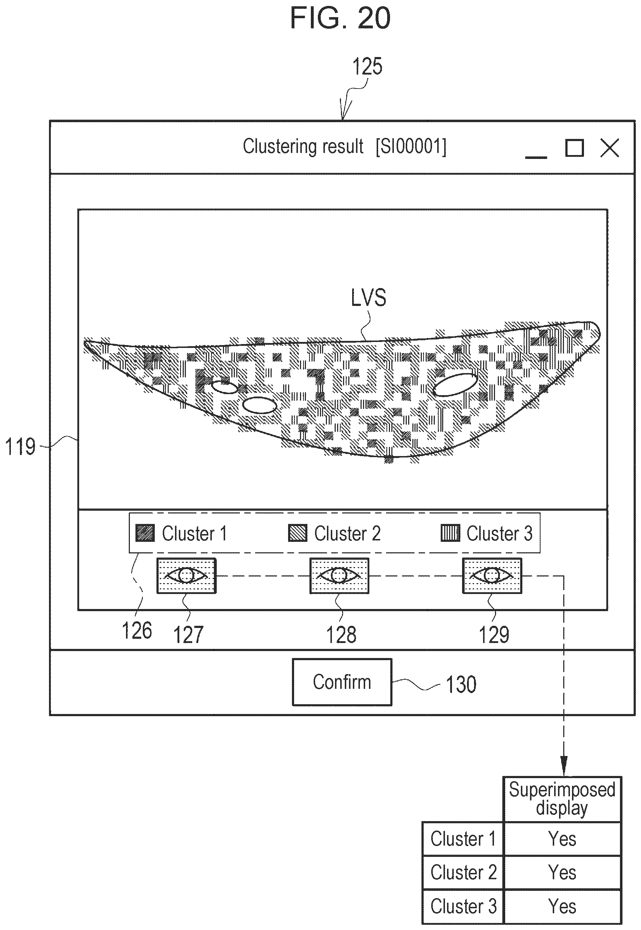

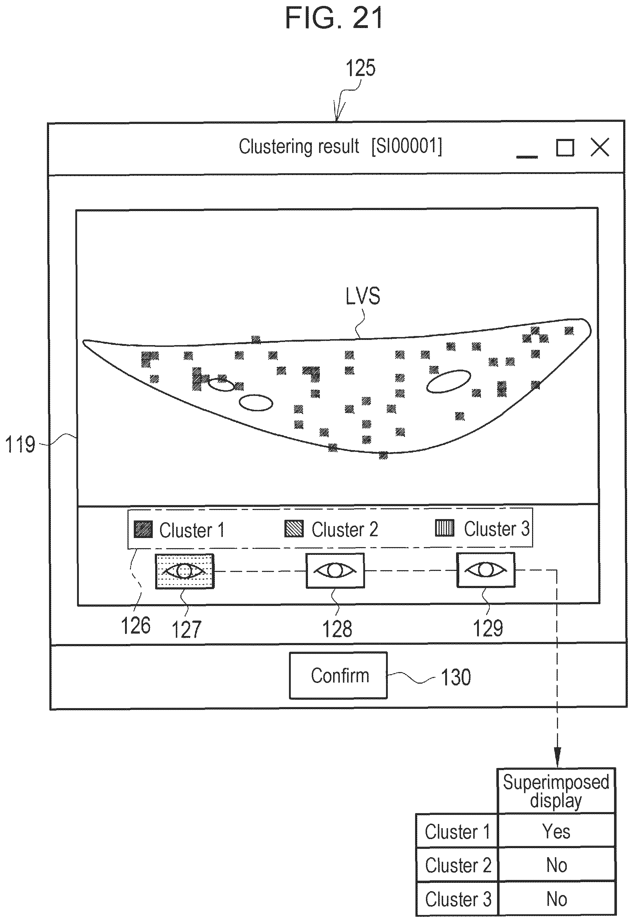

As illustrated in Figs. 20 and 21 as an example, the display control unit 91 performs control to display a clustering result display screen 125 including the superimposed image 119 on the display 12. In addition to the superimposed image 119, the clustering result display screen 125 is provided with a guide 126 for the clusters 1 to 3 and display switching buttons 127, 128, and 129. The display switching button 127 is a button for selecting whether to display the cluster image 115 to be superimposed on the target first specimen image 151T. The display switching button 128 is a button for selecting whether to display the cluster image 116 to be superimposed on the target first specimen image 151T. The display switching button 129 is a button for selecting whether to display the cluster image 117 to be superimposed on the target first specimen image 151T. The designation reception unit 92 receives selection instructions for the respective display switching buttons 127 to 129 from the drug discovery staff member DS.

When the display switching button 127 is selected, the display control unit 91 displays the cluster image 115 to be superimposed on the target first specimen image 151T. On the other hand, when the display switching button 127 is not selected, the display control unit 91 does not display the cluster image 115 to be superimposed on the target first specimen image 151T. Likewise, when the display switching button 128 is selected, the display control unit 91 displays the cluster image 116 to be displayed on the target first specimen image 151T. When the display switching button 128 is not selected, the display control unit 91 does not display the cluster image 116 to be superimposed on the target first specimen image 151T. In addition, when the display switching button 129 is selected, the display control unit 91 displays the cluster image 117 to be superimposed on the target first specimen image 151T. When the display switching button 129 is not selected, the display control unit 91 does not display the cluster image 117 to be superimposed on the target first specimen image 151T.

Therefore, as illustrated in Fig. 20, when all the display switching buttons 127 to 129 are selected, the display control unit 91 displays the superimposed image 119 in which all the cluster images 115 to 117 are superimposed on the target first specimen image 151T. On the other hand, as illustrated in Fig. 21, when the display switching button 127 is selected and the display switching buttons 128 and 129 are not selected, the display control unit 91 displays the superimposed image 119 in which only the cluster image 115 is superimposed on the target first specimen image 151T. As described above, the display control unit 91 displays at least one of the plurality of cluster images 115 to 117 to be superimposed on the target first specimen image 151T. In addition, the designation reception unit 92 receives, from the drug discovery staff member DS, a designation of each of the cluster images 115 to 117 that is displayed to be superimposed on the target first specimen image 151T. Note that the clustering result display screen 125 is initially displayed with all the display switching buttons 127 to 129 selected. In addition, the clustering result display screen 125 is hidden in response to selection of a confirm button 130 at a lower portion thereof.

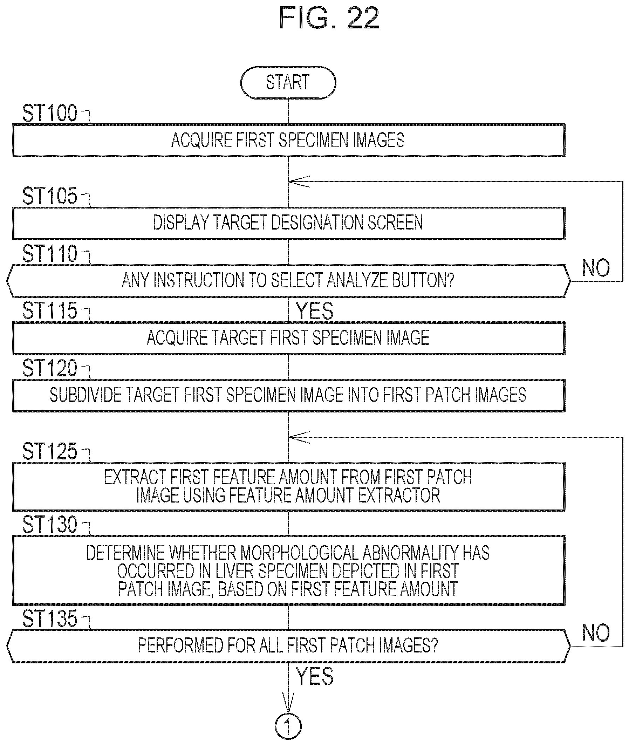

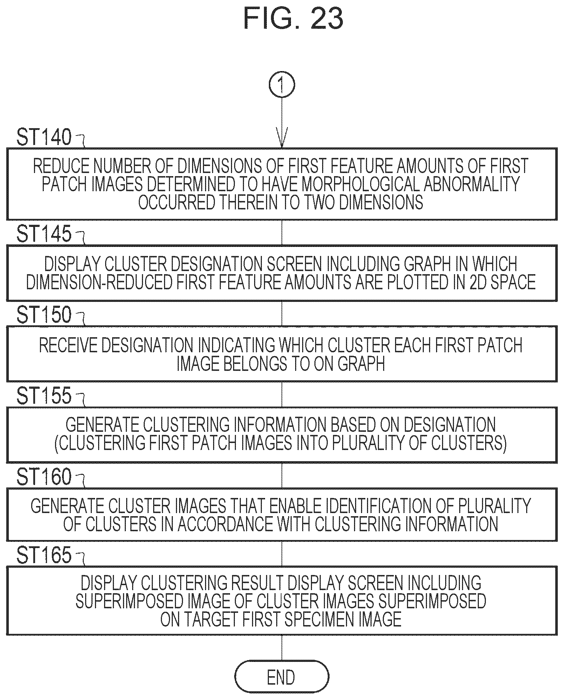

Next, an operation performed by the configuration described above will be described with reference to flowcharts illustrated in Figs. 22 and 23 as an example. First, when the operation program 40 is started in the image processing apparatus 10, the CPU 32 of the image processing apparatus 10 is caused to function as the RW control unit 50, the feature amount extraction unit 51, the determination unit 52, and the clustering processing unit 53 as illustrated in Fig. 3. As illustrated in Fig. 14, the clustering processing unit 53 is caused to function as the dimension reduction unit 90, the display control unit 91, and the designation reception unit 92.

The imaging device 19 captures the first specimen images 151 depicting the liver specimen LVS of the subject S. The first specimen images 151 are output from the imaging device 19 to the image processing apparatus 10. In the image processing apparatus 10, the first specimen images 151 from the imaging device 19 are stored in the storage 30 by the RW control unit 50.

In Fig. 22, when the drug discovery staff member DS inputs a display instruction for the first specimen images 151 through the input device 13, the RW control unit 50 reads out, from the storage 30, and thus acquires the first specimen images 151 designated by the display instruction (step ST100). The first specimen images 151 are output from the RW control unit 50 to the display control unit 91 of the clustering processing unit 53. As illustrated in Fig. 15, the first specimen images 151 are displayed on the display 12 via the target designation screen 95 under the control of the display control unit 91 (step ST105).

When the drug discovery staff member DS places the selection frame 97 at a desired one of the first specimen images 151 in the target designation screen 95 and selects the analyze button 98 (YES in step ST110), the RW control unit 50 reads out and thus acquires the first specimen image 151 at which the selection frame 97 is placed, from the storage 30 as the target first specimen image 151T (step ST115). The target first specimen image 151T is output from the RW control unit 50 to the feature amount extraction unit 51 and the display control unit 91.

The feature amount extractor 41 is read out from the storage 30 by the RW control unit 50, and the read out feature amount extractor 41 is output to the feature amount extraction unit 51. In addition, the second feature amount distribution information 42 is read out and thus acquired from the storage 30 by the RW control unit 50, and the read out second feature amount distribution information 42 is output to the determination unit 52.

As illustrated in Fig. 4, the feature amount extraction unit 51 subdivides the target first specimen image 151T into the plurality of first patch images 651 (step ST120). Subsequently, as illustrated in Fig. 5, the feature amount extraction unit 51 extracts the first feature amount 601 from each of the first patch images 651 using the feature amount extractor 41 (step ST125). The first feature amount 601 is output from the feature amount extraction unit 51 to the determination unit 52.

As illustrated in Fig. 11, the determination unit 52 calculates the distance D between the representative position of the second feature amounts 602 and the position of the first feature amount 601. Then, as illustrated in Figs. 12 and 13, the determination unit 52 compares the distance D with a preset threshold value, and determines whether a morphological abnormality is present in the liver specimen LVS depicted in the first patch image 651 (step ST130). The determination result 61 indicating whether a morphological abnormality is present in the liver specimen LVS depicted in the first patch image 651 is output from the determination unit 52 to the dimension reduction unit 90 of the clustering processing unit 53.

The processing of steps ST125 and ST130 is performed for all the first patch images 651. After the processing of steps ST125 and ST130 is performed for all the first patch images 651 (YES in step ST135), the process proceeds to step ST140 illustrated in Fig. 23.

As illustrated in Fig. 16, the dimension reduction unit 90 reduces the number of dimensions of the first feature amount 601 of each first patch image 651 determined to have a morphological abnormality, to two dimensions (step ST140). Next, under the control of the display control unit 91, the cluster designation screen 105 illustrated in Fig. 17 is displayed on the display 12 (step ST145).

The cluster designation screen 105 includes the graph 106 in which the dimension-reduced two-dimensional first feature amounts 601R are plotted in the two-dimensional feature amount space 107. On this graph 106, as illustrated in Fig. 18, the drug discovery staff member DS designates which cluster each first patch image 651 belongs to, and then selects the complete designation button 109. When the complete designation button 109 is selected, the designation reception unit 92 receives the designation made by the drug discovery staff member DS and indicating which cluster each first patch image 651 belongs to (step ST150). Based on this designation, the designation reception unit 92 generates the clustering information 112. Then, the manual clustering processing of clustering the first patch images 651 into the plurality of clusters is completed (step ST155). The clustering information 112 is output from the designation reception unit 92 to the display control unit 91.

As illustrated in Fig. 19, the display control unit 91 generates the cluster images 115 to 117 that enable identification of the plurality of clusters 1 to 3 by processing the target first specimen image 151T in accordance with the clustering information 112 (step ST160). Then, as illustrated in Figs. 20 and 21, under the control of the display control unit 91, the clustering result display screen 125 including the superimposed image 119 in which at least one of the cluster images 115 to 117 is superimposed on the target first specimen image 151T is displayed on the display 12 (step ST165).

The drug discovery staff member DS views the superimposed image 119 via the clustering result display screen 125 and evaluates the efficacy and toxicity of the candidate substance 11. At this time, the drug discovery staff member DS operates the display switching buttons 127 to 129 if necessary to switch the cluster images 115 to 117 that are displayed to be superimposed on the target first specimen image 151T.

As described above, the CPU 32 of the image processing apparatus 10 includes the RW control unit 50, the feature amount extraction unit 51, the determination unit 52, and the clustering processing unit 53. The RW control unit 50 reads out and thus acquires the target first specimen image 151T from the storage 30. The target first specimen image 151T is an image depicting the liver specimen LVS of the subject S.

The feature amount extraction unit 51 extracts, using the feature amount extractor 41, the first feature amounts 601 from the respective first patch images 651 into which the target first specimen image 151T is subdivided. The determination unit 52 determines, based on each of the first feature amounts 601, whether a morphological abnormality is present in the liver specimen LVS depicted in the corresponding first patch image 651. The clustering processing unit 53 receives, from the drug discovery staff member DS, a designation indicating which cluster the first patch image 651 determined to have a morphological abnormality in the liver specimen LVS belongs to. Then, based on the designation, the clustering processing unit53 performs manual clustering processing of clustering the first patch image 651 into one of the plurality of clusters. Therefore, it becomes possible to cluster the first patch images 651 of the target first specimen image 151T of which the distribution of the first feature amounts 601 is not discrete.

The display control unit 91 performs control to display the superimposed image 119 including the cluster images 115 to 117 that are a result of the manual clustering processing on the display 12. Therefore, the drug discovery staff member DS can easily know the result of the manual clustering processing.

The result of the manual clustering processing is displayed by the plurality of cluster images 115 to 117 generated by processing the target first specimen image 151T. The plurality of cluster images 115 to 117 are images that enable the plurality of clusters 1 to 3 to be identified based on the display format 118 preset for each of the plurality of clusters 1 to 3. Therefore, the drug discovery staff member DS can easily grasp the result of the manual clustering processing.

The display control unit 91 displays at least one of the plurality of cluster images 115 to 117 to be superimposed on the target first specimen image 151T. Therefore, the drug discovery staff member DS can easily grasp which part of the liver specimen LVS belongs to which cluster and, consequently what type of morphological abnormality is present in which part of the liver specimen LVS.

The type of morphological abnormality can be estimated with a certain probability depending on the part where the morphological abnormality is present. Therefore, the drug discovery staff member DS can identify, based on the superimposed image 119, whether the morphological abnormality is caused by the toxicity of the candidate substance 11, such as hyperplasia, or is a natural phenomenon not caused by the toxicity of the candidate substance 11, such as glycogen decrease. Therefore, based on the superimposed image 119, the drug discovery staff member DS can examine a mechanism of action for both the efficacy and toxicity of the candidate substance 11.

The designation reception unit 92 receives, from the drug discovery staff member DS, the designation of the cluster images 115 to 117 displayed to be superimposed on the target first specimen image 151T. Therefore, the drug discovery staff member DS can display only one cluster image of interest to be superimposed on the target first specimen image 151T or display all the cluster images 115 to 117 to be superimposed on the target first specimen image 151T. This expedites the evaluation of the efficacy and toxicity of the candidate substance 11 by the drug discovery staff member DS.

The dimension reduction unit 90 reduces the number of dimensions of the first feature amounts 601 to two dimensions. The display control unit 91 displays, on the display 12, the cluster designation screen 105 including the graph 106 in which the dimension-reduced two-dimensional first feature amounts 601R are plotted in the two-dimensional feature amount space 107. The designation reception unit 92 receives the designation on the graph 106. Therefore, the drug discovery staff member DS can easily designate which cluster each first patch image 651 belongs to.

As illustrated in Figs. 7 and 8, the feature amount extractor 41 is a model trained using, as labeled training data, the second patch images 652 into which the second specimen images 152 are subdivided which depict the liver specimens LVS of the plurality of subjects S constituting the past control group 75 to which the candidate substance for a medicine is not administered in the past evaluation tests of the candidate substance. Therefore, the first feature amount 601 that well represents the characteristics of the shape and texture of the liver specimen LVS can be easily extracted. In addition, the second patch images 652 can be adopted as labeled training data unconditionally without requiring troublesome preprocessing such as a pathologist or the like discriminating whether a morphological abnormality is present in the liver specimen LVS and selectively adopting, as the labeled training data, only the second patch images 652 in which no morphological abnormality is present in the liver specimen LVS. Therefore, a large amount of labeled training data can be easily obtained.

The RW control unit 50 reads out and thus acquires, from the storage 30, the second feature amount distribution information 42 that is information on the distribution 83 of the second feature amounts 602 extracted using the feature amount extractor 41 from the second patch images 652 into which the second specimen images 152 are subdivided. The determination unit 52 calculates the distance D between the distribution 83 and the first feature amount 601, more specifically, the distance D between the representative position of the distribution 83 and the position of the first feature amount, and performs the determination based on the distance D.

The distance D indicates the degree of deviation between the target first specimen image 151T and the second specimen image 152. At least no morphological abnormality due to the toxicity of the candidate substance is present in the liver specimen LVS depicted in the second specimen image 152. Therefore, if the determination is performed based on the distance D, the reasonable determination result 61 can be obtained.

### (Modification)

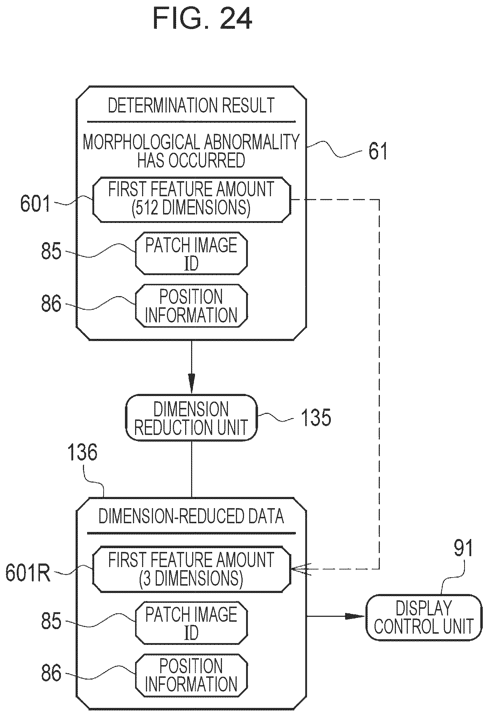

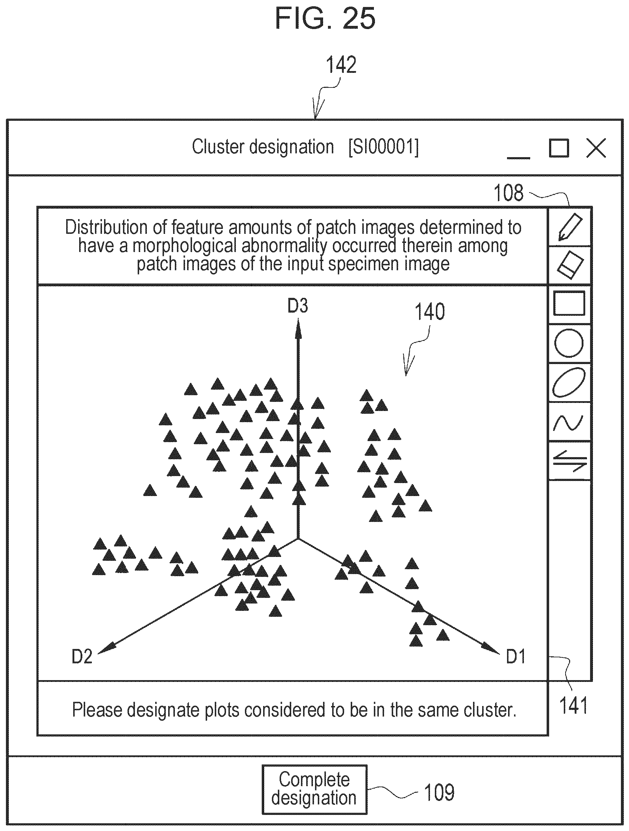

The number of dimensions of the dimension-reduced first feature amount 601R is not limited to the two dimensions illustrated in Fig. 16. For example, just like a dimension reduction unit 135 illustrated in Fig. 24 as an example, dimension-reduced data 136 obtained by reducing the number of dimensions of the first feature amount 601 to three dimensions may be output. In this case, as illustrated in Fig. 25 as an example, the display control unit 91 performs control to display, on the display 12, a cluster designation screen 142 including a graph 141 in which the dimension-reduced first feature amounts 601R are plotted in a three-dimensional feature amount space 140. In this case, a tool for enclosing a group of the plots of the first feature amounts 601R, which is considered to be a cluster, using a cubic, spherical, ellipsoidal, or free curved body. With this modification as well, the drug discovery staff member DS can easily designate which cluster each first patch image 651 belongs to.

### [First Embodiment_2]

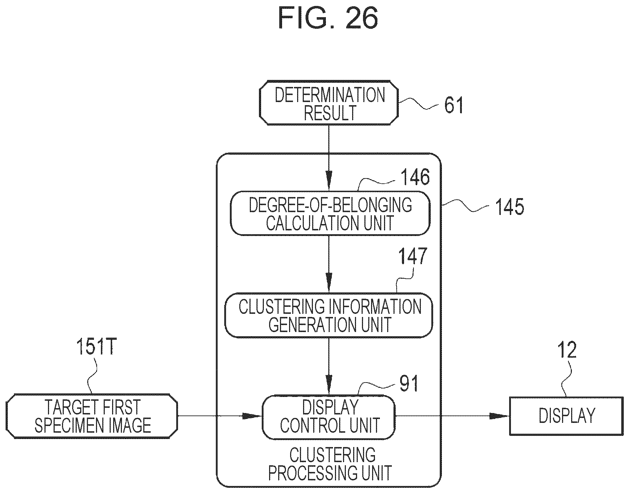

As illustrated in Fig. 26 as an example, a clustering processing unit 145 of the present embodiment includes a degree-of-belonging calculation unit 146, a clustering information generation unit 147, and the display control unit 91. The degree-of-belonging calculation unit 146 performs soft clustering processing described below. The determination result 61 from the determination unit 52 is input to the degree-of-belonging calculation unit 146.

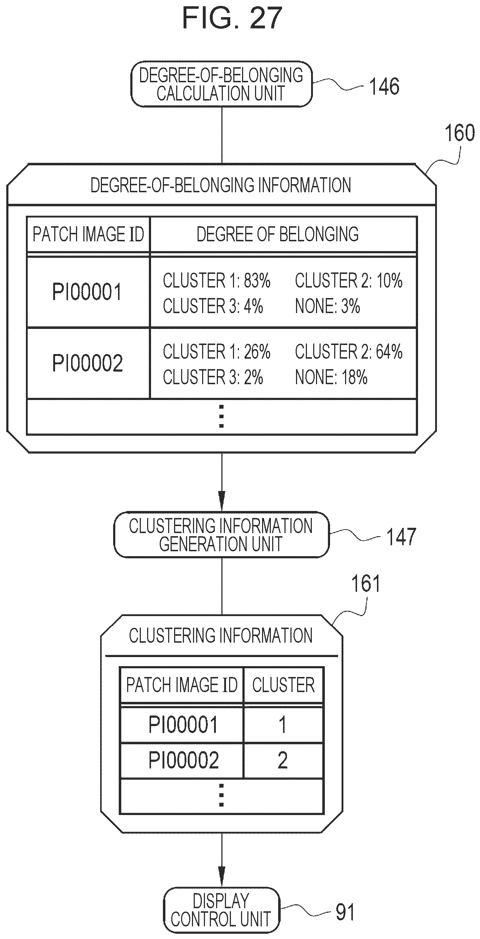

As illustrated in Fig. 27 as an example, the degree-of-belonging calculation unit 146 applies a well known soft clustering method to the first feature amount 601 included in the determination result 61 to calculate the degree of belonging of the first patch image 651 to each cluster. Herein, the degree-of-belonging calculation unit 146 calculates the degrees of belonging to the three clusters (clusters 1 to 3) and a probability of not belonging to any of the clusters 1 to 3 (denoted as "none" in Fig. 27). The sum of the degrees of belonging to the clusters 1 to 3 and the probability of not belonging to any of the clusters 1 to 3 is equal to 100%. As the soft clustering method, a method such as a Gaussian mixture model (GMM), probabilistic latent semantic analysis (PLSA), non-negative matrix factorization (NMF), or fuzzy c-means (FCM) can be used.

The degree-of-belonging calculation unit 146 generates degree-of-belonging information 160 in which the degrees of belonging and the probability of not belonging are registered for each patch image ID 85 of the first patch image 651. The degree-of-belonging calculation unit 146 outputs the degree-of-belonging information 160 to the clustering information generation unit 147.

The clustering information generation unit 147 specifies the cluster to which each of the plurality of first patch images 651 belongs, based on the degree-of-belonging information 160. More specifically, the clustering information generation unit 147 specifies, as the cluster to which the first patch image 651 belongs, the cluster having the largest value among the degrees of belonging registered in the degree-of-belonging information 160. If the probability of not belonging is the largest value, the clustering information generation unit 147 specifies the corresponding first patch image 651 as not belonging to any of the clusters.

The clustering information generation unit 147 generates clustering information 161 representing the specified result. The clustering information 161 is information in which, for each patch image ID 85 of the first patch image 651, the cluster to which the first patch image 651 belongs is registered, similarly to the clustering information 112 of the first embodiment_1. The clustering information generation unit 147 outputs the clustering information 161 to the display control unit 91. Thereafter, the display control unit 91 generates a plurality of cluster images by processing the target first specimen image 151T in accordance with the clustering information 161, as in the first embodiment_1 described above. Then, the display control unit 91 generates a superimposed image in which the cluster images are superimposed on the target first specimen image 151T, and performs control to display a clustering result display screen including the superimposed image on the display 12.

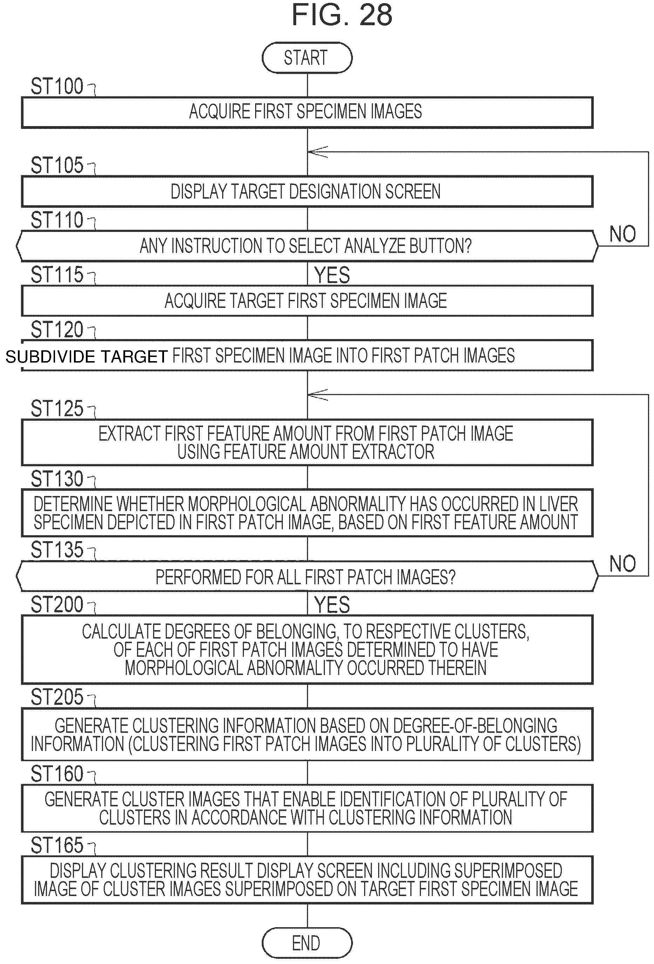

As in a flowchart illustrated in Fig. 28 as an example, in the present embodiment, after the processing of steps ST125 and ST130 is performed for all the first patch images 651 (YES in step ST135), the degree-of-belonging calculation unit 146 of the clustering processing unit 145 calculates the degrees of belonging to the plurality of clusters and the probability of not belonging for the first patch image 651 determined to have a morphological abnormality in the liver specimen LVS (step ST200). The degree-of-belonging calculation unit 146 generates the degree-of-belonging information 160 in which the calculation results of the degrees of belonging and the probability of not belonging are collected for each first patch image 651. The degree-of-belonging information 160 is output from the degree-of-belonging calculation unit 146 to the clustering information generation unit 147. Then, the soft clustering processing is completed.

The clustering information generation unit 147 generates the clustering information 161, based on the degree-of-belonging information 160. As a result, the first patch images 651 are clustered into the plurality of clusters (step ST205). The clustering information 161 is output from the clustering information generation unit 147 to the display control unit 91. Since the subsequent processing is substantially the same as that in the first embodiment_1, the description thereof is omitted.

As described above, the clustering processing unit 145 of the present embodiment performs soft clustering processing to calculate the degree of belonging of the first patch image 651, determined to have a morphological abnormality in the liver specimen LVS, to each of the plurality of clusters. Therefore, even with the present embodiment, it is possible to cluster the first patch images 651 of the target first specimen image 151T of which the distribution of the first feature amounts 601 is not discrete. In addition, since the drug discovery staff member DS does not need to designate which cluster the first patch image 651 belongs to, the burden on the drug discovery staff member DS can be reduced.

As in the first embodiment_1, the degrees of belonging may be calculated by the degree-of-belonging calculation unit 146 after the number of dimensions of the first feature amount 601 is reduced.

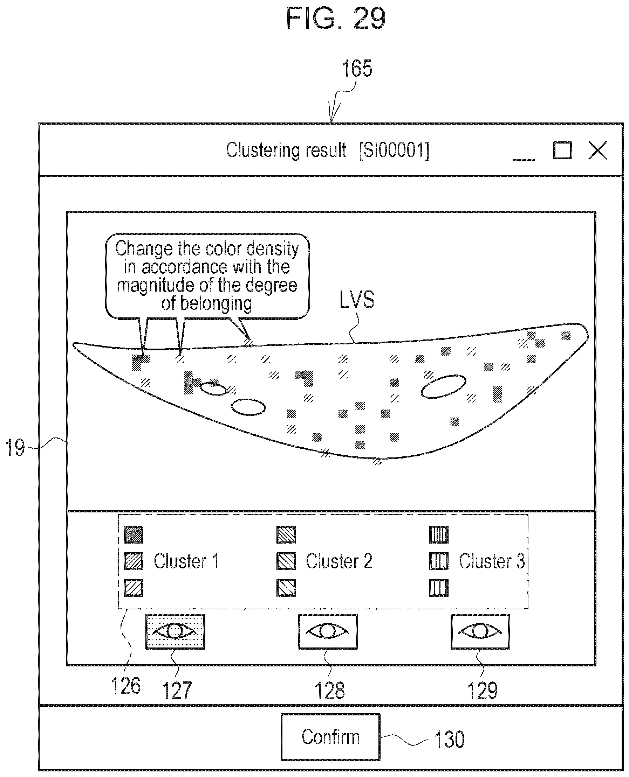

In the present embodiment, when only one cluster image is displayed to be superimposed on the target first specimen image 151T, a display manner such as a clustering result display screen 165 illustrated in Fig. 29 as an example may be employed. That is, in the single cluster image superimposed on the target first specimen image 151T, the color density is changed in accordance with the magnitude of the degree of belonging. Fig. 29 illustrates a case where only the cluster image 115 of the cluster 1 is displayed to be superimposed on the target first specimen image 151T. The case is illustrated where the color density is changed in three levels in accordance with the magnitude of the degree of belonging. The drug discovery staff member DS can easily grasp the magnitude of the degree of belonging by the color density. This further expedites the evaluation of the efficacy and toxicity of the candidate substance 11.

### [Second Embodiment]

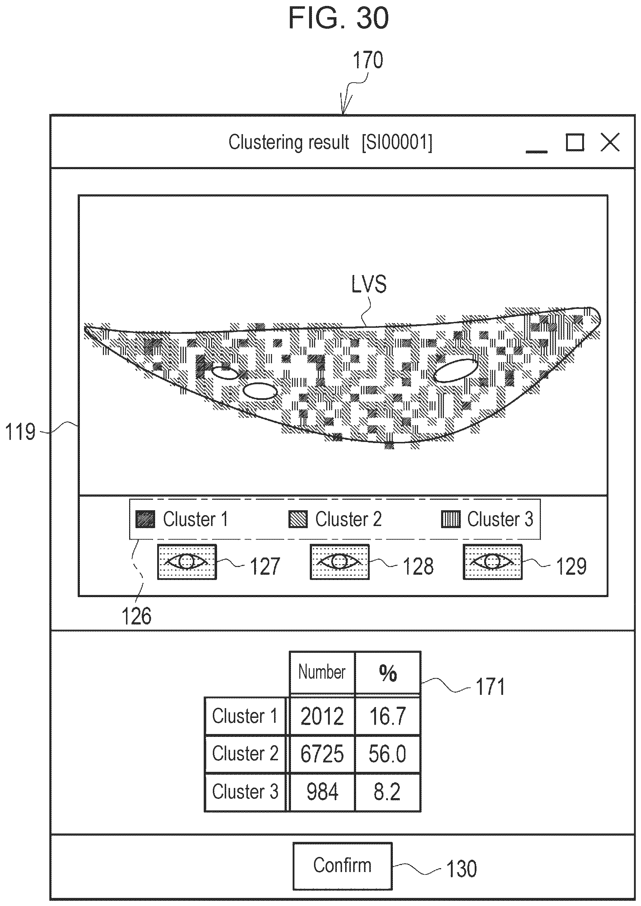

In the present embodiment, as in a clustering result display screen 170 illustrated in Fig. 30 as an example, the display control unit 91 displays statistical information 171 based on the result of the clustering processing. The statistical information 171 presents the number and the percentage of first patch images 651 belonging to each of the clusters 1 to 3. The percentage is calculated by dividing the number of first patch images 651 belonging to each of the clusters 1 to 3 by the total number of first patch images 651.

By thus displaying the statistical information 171, the drug discovery staff member DS can easily grasp the proportion of the first patch images 651 belonging to each of the clusters 1 to 3. In addition to or instead of the number and percentage of first patch images 651 belonging to each of the clusters 1 to 3, the area of the first patch images 651 belonging to each of the clusters 1 to 3 may be displayed as the statistical information.

### [Third Embodiment]

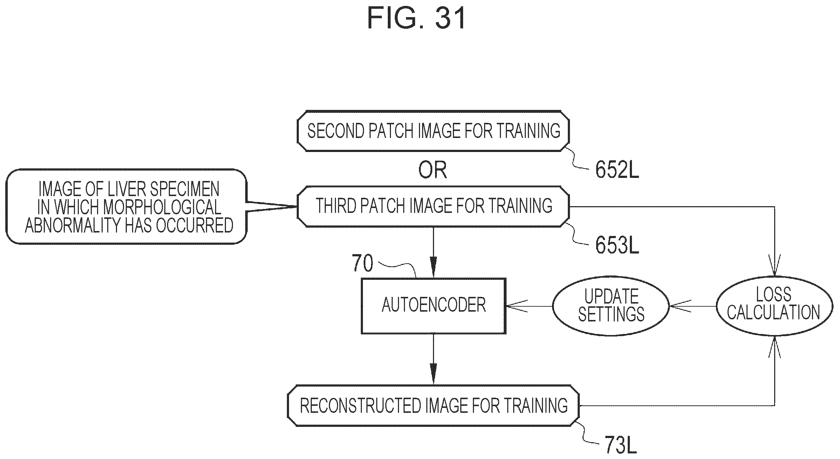

As illustrated in Fig. 31 as an example, in the present embodiment, in addition to the second patch images 652L for training, third patch images 653L for training depicting the liver specimen LVS in which a morphological abnormality is present are also used as labeled training data for the autoencoder 70.

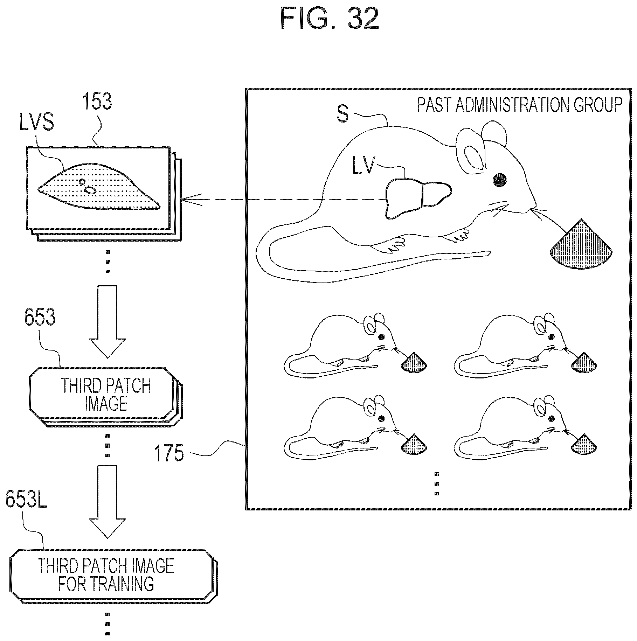

As illustrated in Fig. 32 as an example, the third patch images 653L for training are supplied from among a plurality of third patch images 653 into which third specimen images 153 are subdivided. The third specimen images 153 are images depicting the liver specimens LVS of the subjects S in a past administration group 175. The past administration group 175 is constituted by the plurality of subjects S to which the candidate substance was administered in past evaluation tests. The number of subjects S constituting the past administration group 175 ranges, for example, approximately from several hundreds to several thousands, as in the past control group 75. Since a plurality of third specimen images 153 are obtained from a single subject S just like the first specimen images 151, as many third specimen images 153 as a product of the number of third specimen images 153 obtained from the single subject S and the number of subjects S are obtained from the past administration group 175.

The third patch images 653L for training are obtained by choosing, from among the plurality of third patch images 653, the third patch images 653 depicting the liver specimen LVS in which a morphological abnormality is present. The third patch images 653L for training are chosen manually, for example, by a pathologist or the like. Alternatively, the third patch images 653L for training may be chosen in such a manner that by using the method described in the first embodiment_1,a third feature amount is extracted from the third patch image 653, and it is determined whether a morphological abnormality is present in the liver specimen LVS based on the third feature amount.

As described above, in the third embodiment, not only the second patch images 652L for training but also the third patch images 653L for training depicting the liver specimen LVS in which a morphological abnormality is present are used as labeled training data. Therefore, the autoencoder 70, and consequently the feature amount extractor 41, can learn the liver specimens LVS having more diverse characteristics of the shape and texture. As a result, it is possible to extract the first feature amount 601 that better represents the characteristics of the shape and texture of the liver specimen LVS.

Note that the patch images depicting the liver specimen LVS in which a morphological abnormality is present are not limited to the third patch images 653L for training obtained from the subjects S constituting the past administration group 175 described as an example. A morphological abnormality may also occur in the subjects S constituting the past control group 75. Therefore, it does not matter whether the subject S belongs to the past control group 75 or the past administration group 175, as long as the patch image depicts the liver specimen LVS in which a morphological abnormality is present. That is, the second patch images 652L for training depicting the liver specimen LVS in which a morphological abnormality is present may be used as labeled training data. Further, the patch images depicting the liver specimen LVS in which a morphological abnormality is present may also be images obtained from the subject S intentionally subjected to various stresses to induce such a morphological abnormality. Additionally, the patch images depicting the liver specimen LVS in which a morphological abnormality is present may also be images obtained by processing the patch image depicting a normal liver specimen LVS to artificially create a morphological abnormality.

### [Fourth Embodiment]

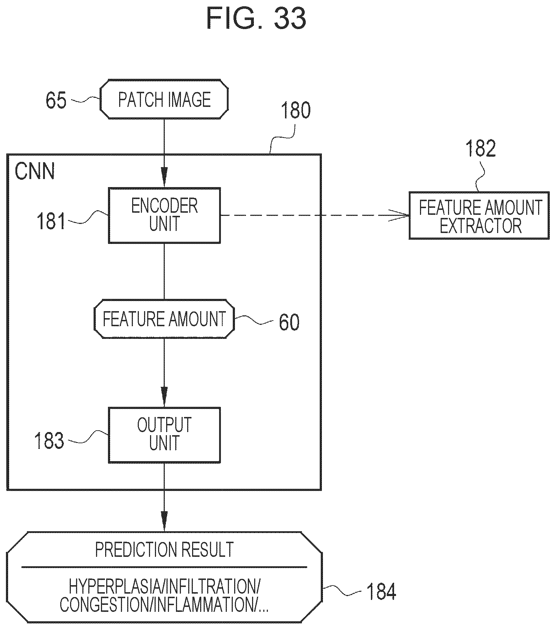

As illustrated in Fig. 33 as an example, in the present embodiment, an encoder unit 181 of a convolutional neural network (hereinafter referred to as CNN) 180 is repurposed as a feature amount extractor 182 for extracting the first feature amount 601 from the first patch image 651. The feature amount extractor 182 is an example of a "machine learning model" according to the technique of the present disclosure.

The CNN 180 has an output unit 183 in addition to the encoder unit 181. The patch image 65, such as the first patch image 651, is input to the encoder unit 181. The encoder unit 181 converts the patch image 65 into the feature amount 60. The encoder unit 181 passes the feature amount 60 to the output unit 183. The output unit 183 outputs a prediction result 184, based on the feature amount 60. The prediction result 184 is a prediction of one type of morphological abnormality that is present in the liver specimen LVS depicted in the patch image 65 from among a plurality of types such as hyperplasia, infiltration, congestion, and inflammation.

In a training phase of the CNN 180, in addition to the second patch images 652L for training, patch images depicting the liver specimen LVS in which a morphological abnormality is present, presented in the third embodiment, are used mainly as labeled training data. As described above, a morphological abnormality may also occur in the subjects S constituting the past control group 75. Therefore, the second patch images 652L for training may also depict the liver specimen LVS in which a morphological abnormality is present. Accordingly, in the training phase, it is preferable to perform label smoothing on ground truth data and add an error rate, rather than strictly classifying the type of the morphological abnormality with 1 or 0. In addition, regions that are likely to be erroneously detected as a morphological abnormality in the liver specimen LVS depicted in the third patch images 653L for training may be masked and then used in training. Examples of the regions that are likely to be erroneously detected as a morphological abnormality include dust or the like adhered when the slide specimen 18 is prepared.

As described above, in the fourth embodiment, the CNN 180, and consequently the feature amount extractor 182, is a model that performs a task of identifying the type of morphological abnormality. Therefore, the first feature amount 601 that still better represents the characteristics of the shape and texture of the liver specimen LVS can be extracted.

Note that the machine learning model to be repurposed as the feature amount extractor is not limited to the autoencoder 70 and the CNN 180 given as an example. A generator of a generative adversarial network (GAN) may also be repurposed as the feature amount extractor. A machine learning model without convolutional layers, such as a Vision Transformer (ViT), may also be repurposed as the feature amount extractor.

Contrastive learning may be performed in which learning is performed to make a distance between feature amounts derived from the same image in the feature amount space smaller and make a distance between feature amounts derived from different images in the feature amount space larger. As the contrastive learning, a learning method such as a simple framework for contrastive learning of visual representations (SimCLR) is known, for example. In addition, a learning method such as Bootstrap Your Own Latent (BYOL), which does not use the aforementioned pairs of different images (also referred to as negative samples), may be used. In addition, constraints may be applied to the distribution of the extracted feature amounts, such as constraining the distribution to be a distribution on a unit sphere or to follow a standard normal distribution.

### [Fifth Embodiment]

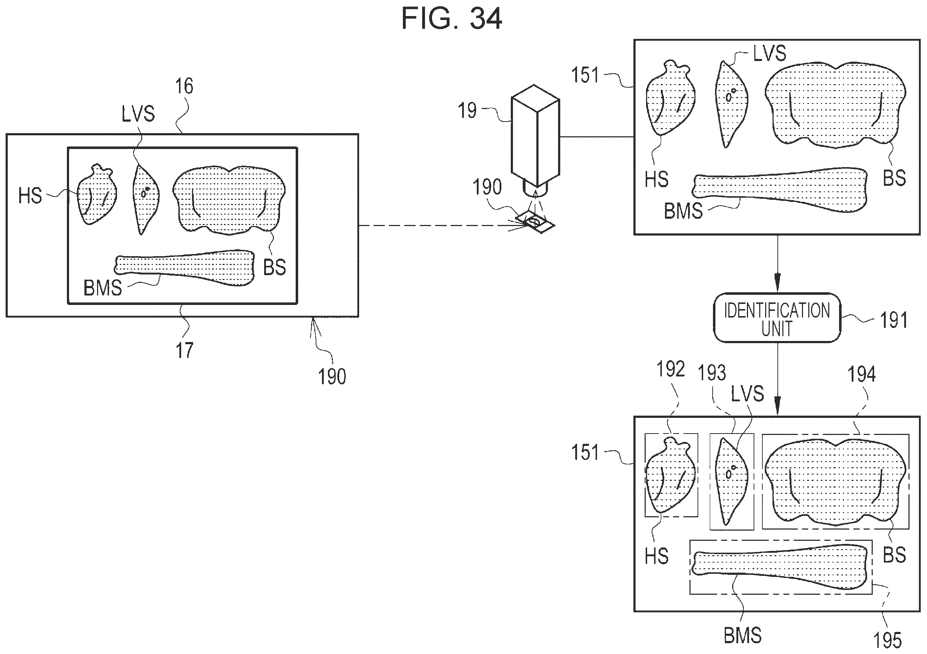

As illustrated in Fig. 34 as an example, in a fifth embodiment, a slide specimen 190 on which tissue specimens of a plurality of types of organs are placed on the single slide glass 16 is handled. Fig. 34 illustrates a case where, in addition to the liver specimen LVS, a heart specimen HS, a brain specimen BS, and a bone marrow specimen BMS are placed. In this case, the first specimen image 151 depicts the heart specimen HS, the brain specimen BS, and the bone marrow specimen BMS in addition to the liver specimen LVS.

The CPU 32 of the image processing apparatus 10 according to the fifth embodiment functions as an identification unit 191, in addition to the processing units 50 to 53 described in the first embodiment above. The identification unit 191 identifies tissue specimens of respective organs from the first specimen image 151 using, for example, templates for identifying the tissue specimens of the respective organs or a machine learning model. The identification unit 191 outputs coordinate information of frames 192 to 195 enclosing the tissue specimens of the respective organs as an identification result. The frame 192 is a frame enclosing the heart specimen HS. The frame 193 is a frame enclosing the liver specimen LVS. In addition, the frame 194 is a frame enclosing the brain specimen BS. The frame 195 is a frame enclosing the bone marrow specimen BMS.

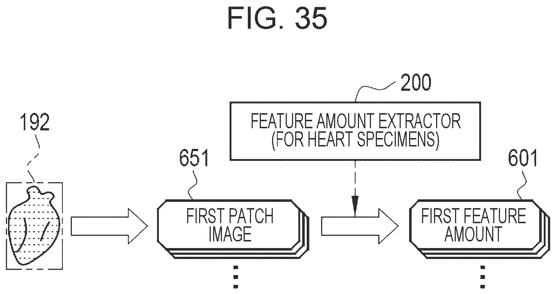

Fig. 35 illustrates how the feature amount extraction unit 51 subdivides the heart specimen HS in the frame 192 into the plurality of first patch images 651 and extracts, using a feature amount extractor 200 for the heart specimens HS, the first feature amounts 601 from the first patch images 651. The feature amount extraction unit 51 also extracts the first feature amounts 601 for the liver specimen LVS in the frame 193, the brain specimen BS in the frame 194, and the bone marrow specimen BMS in the frame 195. That is, the feature amount extraction unit 51 extracts the first feature amounts 601 for each of the tissue specimens of the respective organs identified by the identification unit 191. The subsequent processing of the determination unit 52 is the same as the processing described in the first embodiment_1 and others, except that the determination is performed for each of the tissue specimens of the respective organs depicted in the target first specimen image 151T. Thus, the illustration and description thereof are omitted.

As described above, in the fifth embodiment, the first specimen image 151 is an image obtained by imaging the slide specimen 190 on which tissue specimens of a plurality of types of organs are placed. The identification unit 191 identifies the tissue specimens of the respective organs from such a first specimen image 151. The feature amount extraction unit 51 extracts the first feature amount 601 for each of the tissue specimens of the identified organs. The determination unit 52 performs determination for each of the tissue specimens of the organs depicted in the target first specimen image 151T, based on the first feature amount 601. Therefore, the slide specimen 190 on which the tissue specimens of the plurality of types of organs are placed can be handled. As for the slide specimen, the slide specimen 190 on which tissue specimens of a plurality types of organs are placed as in the present embodiment is more common than the slide specimen 18 on which a tissue specimen of a single organ is placed as in the first embodiment. Therefore, processing that matches the more common operational practices can be performed.

The frame indicating the tissue specimen of each organ in the first specimen image 151 may be defined manually by the drug discovery staff member DS.

The feature amount 60 is not limited to that extracted by the feature amount extractor 41. The feature amount 60 may be an average value, a maximum value, a minimum value, a mode value, or a variance of the pixel values of the patch image 65.

The organ is not limited to the liver LV used as an example. The organ may be a stomach, a lung, a small intestine, a large intestine, or the like. Furthermore, the subject S is not limited to a rat. The subject S may be a mouse, a guinea pig, a gerbil, a hamster, a ferret, a rabbit, a dog, a cat, a monkey, or the like.

The image processing apparatus 10 may be a personal computer installed in a pharmaceutical development facility as described in Fig. 1, or may be a server computer installed in a data center independent of the pharmaceutical development facility.

In a case where the image processing apparatus 10 is a server computer, the first specimen image 151 is transmitted from a personal computer installed in each pharmaceutical development facility to the server computer via a network such as the Internet. The server computer distributes various screens such as the target designation screen 95 to the personal computer in a form of screen data for web distribution created using a markup language such as Extensible Markup Language (XML), for example. The personal computer reproduces the screen to be displayed on a web browser based on the screen data, and displays the screen on a display. In addition, other data description languages such as Javascript (registered trademark) Object Notation (JSON) may be used instead of XML.

The image processing apparatus 10 according to the technique of the present disclosure can be widely used throughout all stages of pharmaceutical development, from the earliest stage of setting a drug discovery target to the final stage of clinical trials.

The hardware configuration of the computer constituting the image processing apparatus 10 according to the technique of the present disclosure can be modified in various ways. For example, the image processing apparatus 10 may be constituted by a plurality of computers that are separate in terms of hardware in order to improve the processing capacity and the reliability. For example, the functions of the feature amount extraction unit 51 and the determination unit 52 and the function of the clustering processing unit 53 or 145 can be distributed between two computers. In this case, the image processing apparatus 10 is constituted by the two computers.

As described above, the hardware configuration of the computer of the image processing apparatus 10 can be appropriately modified according to the required performance, such as the processing capacity, safety, and reliability. Further, not only the hardware but also an application program such as the operation program 40 may be duplicated or stored in a distributed manner across a plurality of storages, in order to ensure the safety and reliability.

In each of the embodiments described above, various processors mentioned below can be used as a hardware structure of the processing units that execute various types of processing such as the RW control unit 50, the feature amount extraction unit 51, the determination unit 52, the clustering processing units 53 and 145, the dimension reduction unit 90, the display control unit 91, the designation reception unit 92, the degree-of-belonging calculation unit 146, the clustering information generation unit 147, and the identification unit 191, for example. The various processors include the CPU 32, which is a general-purpose processor that executes software (the operation program 40) to function as various processing units as described above, a programmable logic device (PLD) such as a field programmable gate array (FPGA), which is a processor having a circuit configuration that can be changed after manufacture, and a dedicated electric circuit such as an application specific integrated circuit (ASIC), which is a processor with a circuit configuration specifically designed to execute particular processing.

One processing unit may be constituted by a single processor from among the various processors or by a combination of two or more processors of the same or different types (for example, a combination of a plurality of FPGAs and/or a combination of a CPU and an FPGA). Additionally, a plurality of processing units may be constituted by a single processor.

As an example in which a plurality of processing units are constituted by a single processor, firstly, there is a form represented by computers such as a client and a server, where one processor is constituted by a combination of one or more CPUs and software and this processor functions as the plurality of processing units. Secondly, there is a form that uses a processor that implements the functions of the entire system including the plurality of processing units, on a single integrated circuit (IC) chip, as represented by a system on chip (SoC) or the like. As described above, the various processing units are constituted by one or more of the aforementioned processors in terms of the hardware structure.

Further, as the hardware structure of these various processors, more specifically, an electric circuit (circuitry) in which circuit elements such as semiconductor elements are combined can be used.

From the above description, the technique described in appendices below can be grasped.

### [Appendix 1]

An image processing apparatus comprising:
a processor configured to:
acquire a first specimen image depicting a tissue specimen of a subject;
extract, using a machine learning model, first feature amounts from respective first patch images into which the first specimen image is subdivided;
determine, based on each of the first feature amounts, whether a morphological abnormality is present in the tissue specimen depicted in a corresponding first patch image of the first patch images; and
perform one of
   manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or
   soft clustering processing of calculating a degree of belonging of the first patch image determined to have the morphological abnormality in the tissue specimen to each of the plurality of clusters.

### [Appendix 2]

The image processing apparatus according to appendix 1, wherein the processor is configured to perform control to display a result of the manual clustering processing or the soft clustering processing.

### [Appendix 3]

The image processing apparatus according to appendix 2, wherein
the result is displayed by a plurality of cluster images generated by processing the first specimen image, and
the plurality of cluster images are images that enable the plurality of clusters to be identified based on a display format preset for each of the plurality of clusters.

### [Appendix 4]

The image processing apparatus according to appendix 3, wherein the processor is configured to display at least one cluster image among the plurality of cluster images to be superimposed on the first specimen image.

### [Appendix 5]

The image processing apparatus according to appendix 4, wherein the processor is configured to receive, from the user, a designation of the at least one cluster image displayed to be superimposed on the first specimen image.

### [Appendix 6]

The image processing apparatus according to any one of appendices 2 to 5, wherein the processor is configured to display statistical information based on the result.

### [Appendix 7]

The image processing apparatus according to any one of appendices 1 to 6, wherein the processor is configured to, in the manual clustering processing:
reduce a number of dimensions of the first feature amounts to two or three dimensions;
display a graph in which the first feature amounts with a reduced number of dimensions are plotted in a two-dimensional space or a three-dimensional space; and
receive the designation on the graph.

### [Appendix 8]

The image processing apparatus according to any one of appendices 1 to 7, wherein the machine learning model is a model trained using, as labeled training data, second patch images into which second specimen images are subdivided, the second specimen images depicting tissue specimens of a plurality of subjects constituting a control group to which a candidate substance for a medicine is not administered in a past evaluation test of the candidate substance.

### [Appendix 9]

The image processing apparatus according to appendix 8, wherein the labeled training data further includes a patch image depicting the tissue specimen in which the morphological abnormality is present.

### [Appendix 10]

The image processing apparatus according to appendix 9, wherein the machine learning model is a model that performs a task of identifying a type of the morphological abnormality.

### [Appendix 11]

The image processing apparatus according to any one of appendices 8 to 10, wherein the processor is configured to:
acquire information on a distribution of second feature amounts extracted using the machine learning model from the second patch images into which the second specimen images are subdivided;
calculate a distance between the distribution and each of the first feature amounts; and
perform the determination based on the distance.

The technique of the present disclosure can also be implemented by appropriately combining the various embodiments and/or various modifications described above. Furthermore, the present disclosure is not limited to the embodiments described above, and various configurations may be obviously adopted without departing from the gist. Further, the technique of the present disclosure encompasses not only a program but also a storage medium that stores the program in a non-transitory manner.

The contents and illustrations presented above are a detailed description of part related to the technique of the present disclosure, and are merely an example of the technique of the present disclosure. For example, the description regarding the configuration, functions, operations, and effects described above is a description about an example of the configuration, functions, operations, and effects of the part related to the technique of the present disclosure. Therefore, needless to say, within the scope not departing from the gist of the technique of the present disclosure, an unnecessary portion may be omitted, a new element may be added, or an element may be replaced in the contents described and illustrated above. Furthermore, to avoid confusion and facilitate understanding of the part related to the technique of the present disclosure, the contents described and illustrated above omit descriptions of common technical knowledge and the like that do not particularly require the description for enabling the implementation of the technique of the present disclosure.

In the present specification, "A and/or B" is synonymous with "at least one of A or B". That is, "A and/or B" may mean only A, only B, or a combination of A and B. In addition, in the present specification, when three or more items are expressed by linked with "and/or", the same concept as "A and/or B" is applied.

All documents, patent applications, and technical standards described in this specification are incorporated herein by reference to the same extent as if individual documents, patent applications, and technical standards were specifically and individually described to be incorporated by reference.

## Claims

1. An image processing apparatus comprising:
a processor configured to:
acquire a first specimen image depicting a tissue specimen of a subject;
extract, using a machine learning model, first feature amounts from respective first patch images into which the first specimen image is subdivided;
determine, based on each of the first feature amounts, whether a morphological abnormality is present in the tissue specimen depicted in a corresponding first patch image of the first patch images; and
perform one of
manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or
soft clustering processing of calculating a degree of belonging of the first patch image determined to have the morphological abnormality in the tissue specimen to each of the plurality of clusters.

2. The image processing apparatus according to claim 1, wherein the processor is configured to perform control to display a result of the manual clustering processing or the soft clustering processing.

3. The image processing apparatus according to claim 2, wherein
the result is displayed by a plurality of cluster images generated by processing the first specimen image, and
the plurality of cluster images are images that enable the plurality of clusters to be identified based on a display format preset for each of the plurality of clusters.

4. The image processing apparatus according to claim 3, wherein the processor is configured to display at least one cluster image among the plurality of cluster images to be superimposed on the first specimen image.

5. The image processing apparatus according to claim 4, wherein the processor is configured to receive, from the user, a designation of the at least one cluster image displayed to be superimposed on the first specimen image.

6. The image processing apparatus according to claim 2, wherein the processor is configured to display statistical information based on the result.

7. The image processing apparatus according to claim 1, wherein the processor is configured to, in the manual clustering processing:
reduce a number of dimensions of the first feature amounts to two or three dimensions;
display a graph in which the first feature amounts with a reduced number of dimensions are plotted in a two-dimensional space or a three-dimensional space; and
receive the designation on the graph.

8. The image processing apparatus according to claim 1, wherein the machine learning model is a model trained using, as labeled training data, second patch images into which second specimen images are subdivided, the second specimen images depicting tissue specimens of a plurality of subjects constituting a control group to which a candidate substance for a medicine is not administered in a past evaluation test of the candidate substance.

9. The image processing apparatus according to claim 8, wherein the labeled training data further includes a patch image depicting the tissue specimen in which the morphological abnormality is present.

10. The image processing apparatus according to claim 9, wherein the machine learning model is a model that performs a task of identifying a type of the morphological abnormality.

11. The image processing apparatus according to claim 8, wherein the processor is configured to:
acquire information on a distribution of second feature amounts extracted using the machine learning model from the second patch images into which the second specimen images are subdivided;
calculate a distance between the distribution and each of the first feature amounts; and
perform the determination based on the distance.

12. An operation method of an image processing apparatus, the operation method comprising:
acquiring a first specimen image depicting a tissue specimen of a subject;
extracting, using a machine learning model, first feature amounts from respective first patch images into which the first specimen image is subdivided;
determining, based on each of the first feature amounts, whether a morphological abnormality is present in the tissue specimen depicted in a corresponding first patch image of the first patch images; and
performing one of
manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or
soft clustering processing of calculating a degree of belonging of the first patch image determined to have the morphological abnormality in the tissue specimen to each of the plurality of clusters.

13. An operation program of an image processing apparatus, the operation program causing a computer to execute a process, the process comprising:
acquiring a first specimen image depicting a tissue specimen of a subject;
extracting, using a machine learning model, first feature amounts from respective first patch images into which the first specimen image is subdivided;
determining, based on each of the first feature amounts, whether a morphological abnormality is present in the tissue specimen depicted in a corresponding first patch image of the first patch images; and
performing one of
manual clustering processing of receiving, from a user, a designation indicating which cluster a first patch image determined to have the morphological abnormality in the tissue specimen among the first patch images belongs to, and clustering, based on the designation, the first patch image into one of a plurality of clusters, or
soft clustering processing of calculating a degree of belonging of the first patch image determined to have the morphological abnormality in the tissue specimen to each of the plurality of clusters.
